# FASCICULE DE BREVET EUROPEEN

(11) **EP 2 854 905 B1**
(45) Date de publication et mention de la délivrance du brevet: **28.03.2018**
(21) Numéro de dépôt: 13733338.1
(22) Date de dépôt: 24.05.2013
(51) Int. Cl.: A61M 5/315, A61M 5/32, A61M 5/20, A61M 5/31, A61M 39/10

(54) **AUTOINJECTEUR.**
AUTOINJEKTOR
AUTOINJECTOR

(30) Priorité: 25.05.2012 FR 1254849; 27.09.2012 FR 1259097; 21.03.2013 FR 1352517
(43) Date de publication de la demande: 08.04.2015
(73) Titulaire: Aptar France SAS, 27110 Le Neubourg (FR)
(72) Inventeur: FABIEN, David, F-22700 Saint Quay Perros (FR); MANSENCAL, Antoine, F-33210 Langon (FR); WALTER, Matthieu, F-78110 Le Vesinet (FR)
(74) Mandataire: CAPRI
(86) Numéro de dépôt international: PCT/FR2013/051154
(87) Numéro de publication internationale: WO 2013/175148

(56) Documents cités:
- EP-A1- 2 399 628
- WO-A1-2009/040604
- WO-A1-2010/108116
- WO-A2-2011/048422
- US-A1- 2008 281 271

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois tous un certain nombre d'inconvénients.

Ainsi, pour éviter un déclenchement intempestif de l'autoinjecteur, par exemple pendant un transport ou pendant le stockage, les dispositifs doivent comporter des moyens de verrouillage fiables. De même, lorsqu'un utilisateur souhaite utiliser l'autoinjecteur et qu'il déverrouille le dispositif, par exemple en retirant le capot, le dispositif ne doit pas se déclencher de manière intempestive mais seulement au moment où l'utilisateur le souhaite réellement, c'est-à-dire au moment où il l'applique contre la partie du corps dans laquelle il veut réaliser l'injection. Or, notamment quand les personnes utilisant l'autoinjecteur sont des personnes âgées ou handicapées, il peut arriver que l'utilisateur laisse tomber le dispositif au moment où il souhaite l'utiliser. Il est souhaitable que dans un tel cas l'autoinjecteur ne se déclenche pas tout seul. Il est donc important de prévoir une serrure de déclenchement fiable. D'un autre côté, il ne faut pas que l'utilisation de l'autoinjecteur devienne trop difficile, ce qui empêcherait des personnes faibles de l'utiliser. Il est donc difficile de trouver le bon compromis entre la sécurité du verrouillage et la facilité d'utilisation et d'actionnement de l'autoinjecteur. C'est un des objectifs de la présente invention de répondre à ce problème.

Par ailleurs, selon le volume du produit fluide distribué lors de l'injection et aussi en fonction de sa viscosité, le temps nécessaire pour réaliser cette injection peut être assez important, pouvant notamment dépasser plusieurs secondes. Il est alors très important que l'utilisateur ne retire pas le dispositif de son corps avant que l'injection soit complète. Il est donc souhaitable que le dispositif comporte des moyens permettant d'indiquer de manière fiable à l'utilisateur que l'injection est terminée.

Il est aussi important de garantir que le produit soit injecté à la bonne profondeur dans le corps, c'est-à-dire dans le bon tissu. La maitrise du début d'injection, pour garantir que celle-ci ne commencera que lorsque l'aiguille aura atteint sa position de piquage définitive, est donc aussi un aspect important.

De plus, afin d'éviter tout risque de blessures après l'utilisation du dispositif, l'autoinjecteur doit comprendre un dispositif de sécurité aiguille qui évite que l'aiguille reste apparente après l'utilisation du dispositif. Ce dispositif de sécurité doit évidemment également être fiable et ne pas se libérer trop facilement. Il doit aussi être fonctionnel même si l'utilisateur actionne mal l'autoinjecteur, par exemple s'il le retire trop tôt de son corps, avant la fin de l'injection.

Un autre aspect important avec les autoinjecteurs, notamment lorsque le volume de produit fluide est relativement important et/ou lorsque le produit fluide injecté est relativement visqueux, est de permettre au produit de diffuser du site d'injection pendant quelques secondes après ladite injection. Si l'utilisateur retire l'autoinjecteur immédiatement après la fin de l'injection, une partie du produit peut ressortir du corps de l'utilisateur ce qui diminue l'efficacité du traitement. Il est donc souhaitable de prévoir que l'utilisateur maintienne l'autoinjecteur contre son corps encore pendant quelques secondes après la fin de l'injection. Cet aspect est généralement résolu par les autoinjecteurs existants par la notice d'utilisation qui demande à l'utilisateur de compter dans sa tête un certain nombre de secondes avant de retirer le dispositif. Ceci n'est pas fiable et donc insatisfaisant, car le système dépend alors de l'utilisateur lui-même, qui dans certains cas peut être perturbé ou affaibli par l'action d'injection qu'il vient de réaliser.

Les documents WO2012045833, EP1743666, WO2009095701, WO2012022810, EP2399632, FR2884722, WO9632974, WO2012000832, US2008281271, WO2009040602, WO2009040604, WO2009040607, WO2010108116, WO2011048422, EP2399628, WO2008112472, WO2011101380, WO2011101382, US2005273055, FR2905273, WO2009062508, WO2009037141 et GB2463034, décrivent des dispositifs de l'état de la technique.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui soit fiable d'utilisation, qui permette de garantir la distribution de la totalité du produit fluide à l'endroit souhaité, qui permet à l'utilisateur de déterminer quand il doit retirer ou quand il peut retirer l'autoinjecteur de son corps après son utilisation, qui soit sûr et qui empêche tout risque de blessure, et qui soit simple et peut coûteux à fabriquer et à assembler.

La présente invention a donc pour objet un autoinjecteur tel que décrit dans la revendication 1. Des modes de réalisation avantageux sont décrits dans les revendications dépendantes.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
La figure 1 est une vue schématique en perspective éclatée des composants d'un autoinjecteur, selon un premier mode de réalisation avantageux,
Les figures 2a à 2f sont des vues schématiques en section transversale illustrant les différentes séquences d'utilisation de l'autoinjecteur de la figure 1,
Les figures 3a à 3c illustrent plus précisément trois stades différents d'un manchon actionneur avantageux, respectivement avant, en cours et après utilisation,
La figure 4 est une vue de détail montrant le manchon actionneur dans la position de la figure 3a,
Les figures 5 et 6 sont des vues schématiques en section transversale selon deux plans de coupe différents montrant le manchon actionneur dans la position de la figure 4,
Les figures 7 et 8 sont des vues schématiques en perspective partiellement découpées représentant le manchon actionneur dans la position des figures 5 et 6,
La figure 9 est une vue similaire à celle de la figure 4, en début d'actionnement de l'autoinjecteur, pendant la phase de piquage,
Les figures 10 à 11 sont des vues similaires aux figures 5 et 6, dans la position de la figure 9,
La figure 12 est une vue similaire à celle de la figure 8, dans la position des figures 10 et 11,
La figure 13 est une vue similaire à celle des figures 4 et 9 en cours d'actionnement, en phase d'injection,
Les figures 14 et 15 sont des vues similaires à celles des figures 10 et 11 représentant la position de la figure 13,
La figure 16 est une vue similaire à celle de la figure 12, représentant la position des figures 14 et 15,
La figure 17 est une vue similaire à celle de la figure 13, en fin d'actionnement, lorsque l'utilisateur retire l'autoinjecteur du site d'injection,
La figure 18 est une vue similaire à celle de la figure 17, lorsque le manchon actionneur est verrouillé,
La figure 19 est une vue schématique en perspective éclatée illustrant une serrure d'injection avantageuse,
La figure 20 est une vue schématique en section transversale de la serrure d'injection de la figure 19, en position de blocage,
La figure 21 est une vue similaire à celle de la figure 20, en position de déblocage,
La figure 22 est une vue schématique de dessus en section horizontale de la serrure d'injection de la figure 19, en position de blocage,
La figure 23 est une vue schématique en perspective partiellement découpée de la serrure d'injection de la figure 19, en position de blocage,
La figure 24 est une vue schématique en section transversale de la serrure d'injection de la figure 19, en position de blocage,
La figure 25 est une vue similaire à celle de la figure 23, en position de déblocage,
La figure 26 est une vue similaire à celle de la figure 24 en position de déblocage,
La figure 27 est une vue schématique en perspective éclatée d'un dispositif retardateur avantageux,
La figure 28 est une vue schématique en section transversale du dispositif retardateur de la figure 27 avant son actionnement,
La figure 29 est une vue schématique en section selon la ligne de coupe X de la figure 28,
La figure 30 est une vue schématique en section selon la ligne de coupe Y de la figure 28,
La figure 31 est une vue similaire à celle de la figure 28, en fin d'actionnement du dispositif retardateur,
La figure 32 est une vue schématique en perspective éclatée d'un mécanisme de déplacement de seringue avantageux,
Les figures 33 à 35 sont des vues schématique en perspective partiellement découpées du mécanisme de déplacement de la figure 32, avant actionnement, selon trois orientations différentes,
Les figures 36 et 37 sont des vues similaires aux figures 33 et 35, en cours d'actionnement du mécanisme de déplacement,
Les figures 38 à 41 sont des vues schématiques partielles en perspective partiellement découpées du mécanisme de déplacement de la figure 32, lorsque l'aiguille de la seringue a atteint sa position d'injection dans le corps de l'utilisateur,
Les figures 42 et 43 sont des vues schématiques du mécanisme de déplacement de la figure 32 en début de rétractation initiée par le retardateur,
La figure 44 est une vue schématique du mécanisme de déplacement de la figure 32 en début de rétractation initiée par le manchon actionneur,
Les figures 45 et 46 sont des vues similaires aux figures 43 et 44, en fin d'injection,
La figure 47 est une vue schématique en perspective éclatée des composants d'un autoinjecteur, selon un second mode de réalisation avantageux,
Les figures 48a à 48e sont des vues schématiques en section transversale illustrant les différentes séquences d'utilisation de l'autoinjecteur de la figure 47,
Les figures 49a et 49b sont des vues schématiques en perspective illustrant le corps inférieur et le manchon actionneur de l'autoinjecteur de la figure 47,
Les figures 50a, 50b et 50c illustrent schématiquement la coopération entre le manchon actionneur et le corps inférieur de l'autoinjecteur de la figure 47, respectivement en position avant actionnement, après actionnement mais avant injection et après injection,
La figure 51 est une vue similaire à celle de la figure 50a, illustrant une variante de réalisation,
La figure 52 est une vue de détail agrandie en perspective découpée, montrant une variante de manchon actionneur avec des ponts sécables,
Les figures 53a et 53b illustrent des vues schématiques de l'autoinjecteur avant injection,
Les figures 54a et 54b sont des vues similaires aux figures 53a et 53b, après injection,
La figure 55 est une vue schématique en perspective d'un dispositif d'indication sonore et/ou tactile selon une variante avantageuse,
La figure 56 est une vue similaire à la figure 55, partiellement en section,
Les figures 57a, 57b et 57c montrent l'autoinjecteur avant injection,
Les figures 58a, 58b et 58c montrent l'autoinjecteur après injection mais avant actionnement du dispositif d'indication sonore et/ou tactile,
Les figures 59a, 59b et 59c montrent l'autoinjecteur après injection et après actionnement du dispositif d'indication sonore et/ou tactile,
La figure 60 est une vue schématique en perspective éclatée illustrant une variante de réalisation du dispositif d'indication sonore et/ou tactile,
Les figures 61 et 62 sont des vues schématiques du manchon de commande du dispositif d'indication sonore et/ou tactile de la figure 61,
Les figures 63a et 63b sont des vues schématiques de la clé du dispositif d'indication sonore et/ou tactile de la figure 61,
Les figures 64a, 64b et 64c sont des vues schématiques de l'autoinjecteur de la figure 60, respectivement avant déverrouillage de la serrure d'injection, après déverrouillage de la serrure d'injection et en fin d'injection, illustrant le dispositif d'indication sonore et/ou tactile de la figure 61,
La figure 65 montre une autre variante de réalisation du dispositif d'indication sonore et/ou tactile,
Les figures 66a et 66b sont des vues schématiques de la pastille de support du dispositif d'indication sonore et/ou tactile de la figure 65,
Les figures 67a et 67b sont des vues schématiques de la clé du dispositif d'indication sonore et/ou tactile de la figure 65,
Les figures 68a, 68b et 68c sont des vues similaires aux figures 64a, 64b et 64c, illustrant le dispositif d'indication sonore et/ou tactile de la figure 65,
Les figures 69, 70 et 71 sont des vues de détail des figures 68b et 68c,
La figure 72 montre schématiquement une variante de réalisation du manchon actionneur,
La figure 73 montre une autre vue de la variante de réalisation de la figure 72, et
Les figures 74a, 74b et 74c sont des vues similaires aux figures 50a, 50b et 50c, illustrant la variante de réalisation du manchon actionneur des figures 71 et 72.

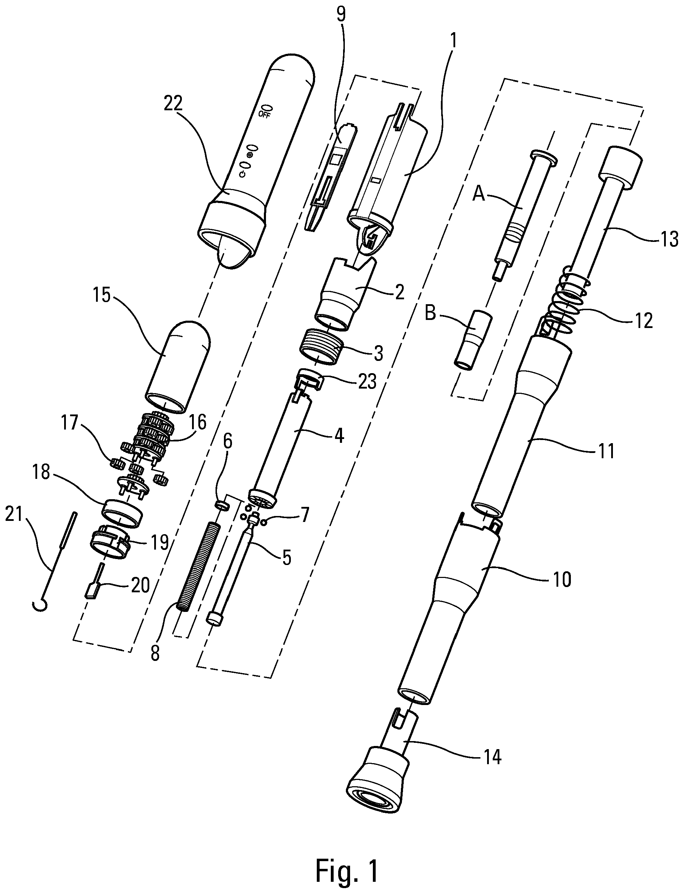

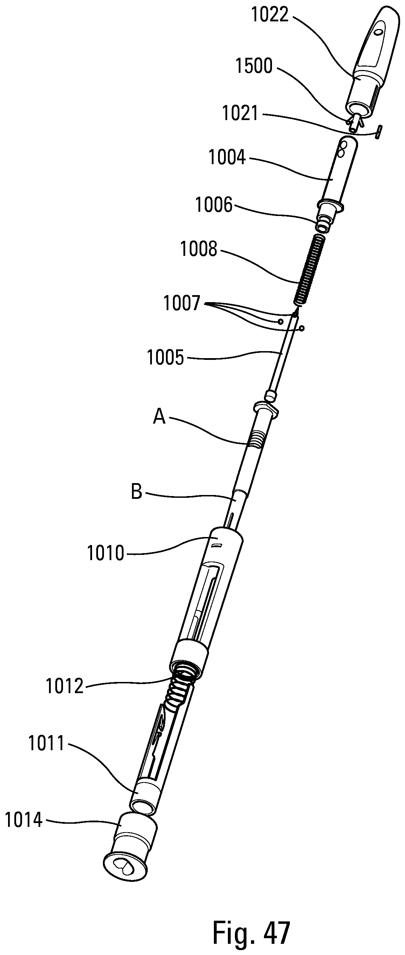

L'autoinjecteur va être décrit ci-après en référence à diverses variantes de deux modes de réalisation avantageux de celui-ci. Un premier mode de réalisation est représenté sur les figures 1 à 46 et un second mode de réalisation est représenté sur les figures 47 à 74c. Il est toutefois à noter que ces autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins.

En se référant à la figure 1, les différents composants de l'autoinjecteur, selon un premier mode de réalisation avantageux, sont représentés de manière éclatée. Dans ce premier mode de réalisation, et dans l'ordre des références numériques, l'autoinjecteur comporte un corps central 1, une bague de commande 2, un ressort de piquage 3, un manchon de commande 4, une tige de piston 5, une pastille support 6, trois éléments de blocage 7, ici sous forme de billes, un ressort d'injection 8, un coulisseau de commande 9, un corps inférieur 10, un manchon actionneur 11, un ressort 12 du manchon actionneur, un logement de réservoir 13, un capot 14, un corps supérieur 15, une pluralité de planétaires 16, une pluralité de satellites 17, un ressort retardateur 18, un déclencheur 19, un doigt de verrouillage 20, un fil 21, une coque externe 22 et une bague de blocage 23. Tous ces éléments font partie du mode de réalisation décrit, mais tous ne sont pas indispensables au fonctionnement de l'autoinjecteur, comme cela sera plus précisément décrit ci après.

Le capot 14 permet notamment de verrouiller l'autoinjecteur pendant le transport et le stockage. Tant que ce capot est assemblé sur le corps inférieur 10, il empêche tout actionnement du manchon actionneur 11, et donc tout déclenchement de l'autoinjecteur.

Un réservoir A peut être insérée dans ledit autoinjecteur. Ce réservoir contient du produit fluide, et comporte un piston et une aiguille. Le piston est adapté à se déplacer dans ledit réservoir pour injecter le produit fluide à travers ladite aiguille. La présente description sera faite en référence à une seringue A, qui peut être de tout type. Plus généralement, il est entendu que le terme « seringue » dans la présente description englobe tout type de réservoir associé à une aiguille.

De préférence, la seringue A est une seringue pré-remplie. Elle comporte avantageusement un capuchon d'aiguille B qui protège et isole l'aiguille avant utilisation de l'autoinjecteur. Avantageusement, ce capuchon d'aiguille B est retiré automatiquement au moment du retrait du capot 14 à partir du corps inférieur 10.

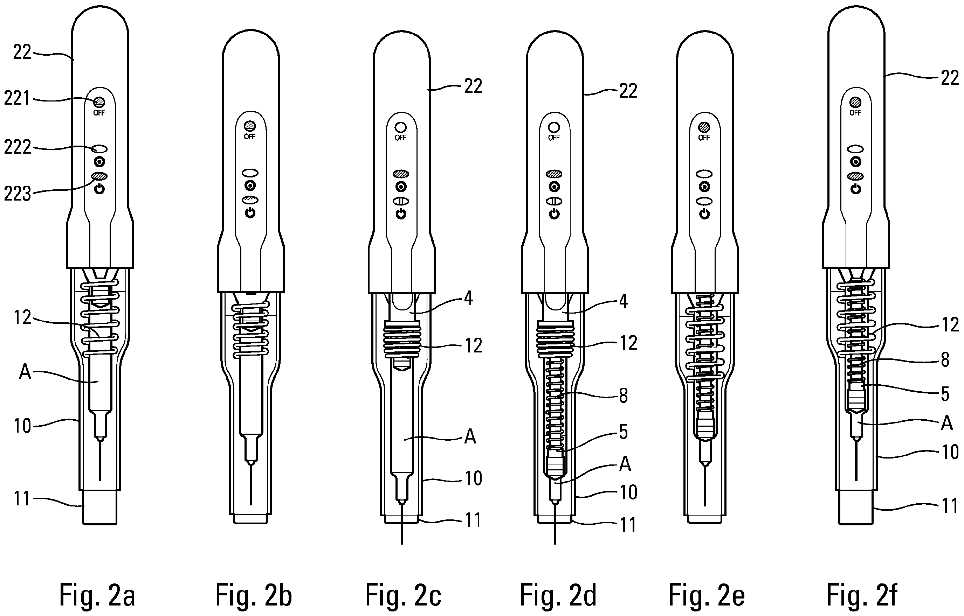

Les figures 2a à 2f illustrent les séquences de l'utilisation de l'autoinjecteur de la figure 1.

Sur la figure 2a, l'autoinjecteur est en position de repos avant utilisation, le capot 14 ayant été retiré.

Lorsque l'utilisateur veut utiliser l'autoinjecteur, il prend le dispositif, par exemple au niveau de la coque externe 22 et il appuie le manchon actionneur 11, qui dans une première position projetée fait saillie hors du corps inférieur 10, contre la partie du corps où il veut réaliser l'injection. Sur la figure 2b, on voit que la pression exercée par l'utilisateur sur le manchon actionneur 11 provoque le coulissement de celui-ci vers l'intérieur du corps inférieur 10, avec pour effet la compression du ressort du manchon actionneur 12.

Lorsque le manchon actionneur 11 atteint sa position d'actionnement, qui est sa position d'extrémité à l'intérieur du corps inférieur 10, il provoque le déclenchement de la serrure de piquage et donc le déplacement du manchon de commande 4 dans le corps inférieur 10 sous l'effet du ressort de piquage 3, avec comme conséquence un déplacement de la seringue A dans le corps inférieur 10 et donc l'insertion de l'aiguille de la seringue dans le corps de l'utilisateur, comme visible sur la figure 2c.

Lorsque l'aiguille atteint sa position d'injection avec une insertion complète de l'aiguille, la phase d'injection est déclenchée, ce qui est représenté sur les figures 2c et 2d. On constate que la tige de piston 5 coulisse à l'intérieur de la seringue A en poussant le piston de celle-ci sous l'effet du ressort d'injection 8. Le produit est donc distribué.

A la fin de l'injection, et avec éventuellement un certain retard ou décalage temporel comme cela sera décrit ultérieurement, l'autoinjecteur prévoit une rétractation de la seringue A. L'aiguille est donc rétractée hors du corps de l'utilisateur vers l'intérieur de l'autoinjecteur, comme représenté sur la figure 2e.

En fin de rétractation, le manchon actionneur 11 est à nouveau déplacé hors du corps inférieur 10 vers une seconde position projetée, sous l'effet du ressort 12 du manchon actionneur, avec un verrouillage dudit manchon actionneur 11, ce qui garantit une sécurité absolue pour l'utilisateur et évite tout risque de blessures avec l'aiguille après utilisation du dispositif. Il est à noter que les première et seconde positions projetées du manchon actionneur, qui, dans l'exemple représenté, sont des positions différentes, pourraient éventuellement être identiques.

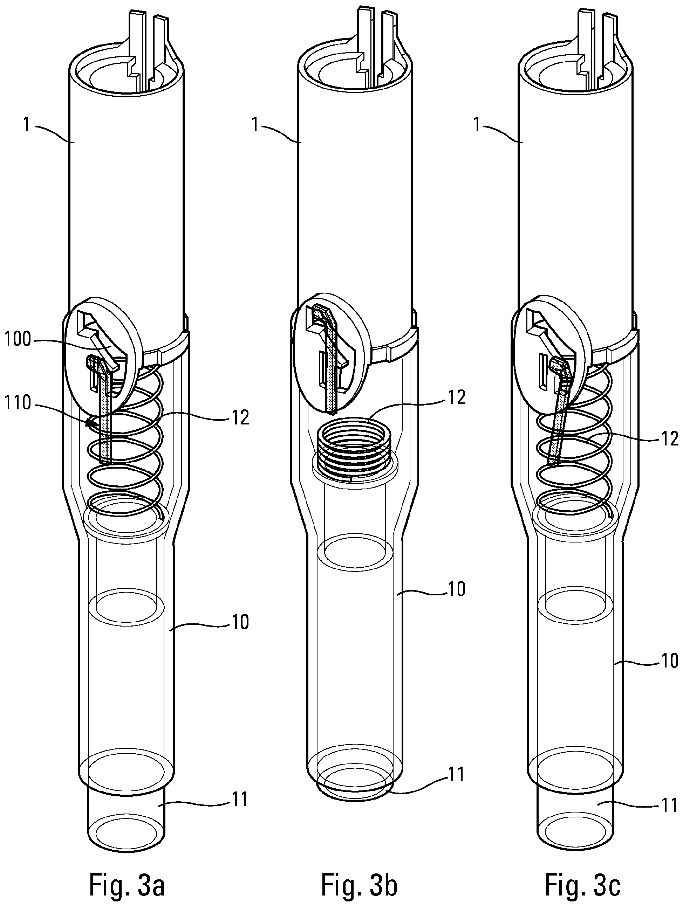

Un manchon actionneur avantageux sera décrit ci-après plus en détail en référence aux figures 3a à 18.

Ledit manchon actionneur 11 comporte une patte flexible 110 qui présente une double flexibilité. Elle est d'une part flexible radialement c'est-à-dire qu'elle se déforme vers l'intérieur du manchon actionneur 11. Elle est ensuite également flexible latéralement c'est-à-dire qu'elle se déforme dans la direction périphérique du manchon actionneur 11. Un manchon actionneur 11 pourvu d'une telle patte flexible est simple à mouler, ce qui est favorable du point de vue des coûts de fabrication. La patte flexible 110 comporte avantageusement une partie de tige 111 qui est flexible et qui se termine par une partie de tête 112. Ladite patte flexible 110 est adaptée à se déformer d'une part radialement et d'autre part latéralement par rapport audit corps central 1 lorsque ledit manchon actionneur 11 est déplacé de sa première position projetée vers sa position d'actionnement puis de sa position d'actionnement en retour vers sa seconde position projetée. De préférence, ladite patte flexible 110 est déformée radialement lorsque ledit manchon actionneur 11 se déplace de sa première position projetée, avant actionnement, vers sa position d'actionnement, et ladite patte flexible est déformée latéralement lorsque ledit manchon actionneur 11 se déplace de sa position d'actionnement vers sa seconde position projetée, en fin d'utilisation. C'est cette variante qui est représentée sur les figures.

Les figures 3a, 3b et 3c sont trois vues schématiques partielles en perspective qui montrent les positions d'extrémités du manchon actionneur 11, à savoir sur la figure 3a la première position projetée au repos avant actionnement, sur la figure 3b la position d'actionnement dans laquelle le manchon actionneur 11 a été inséré au maximum à l'intérieur du corps inférieur 10, et sur la figure 3c la seconde position projetée avec le manchon actionneur 11 verrouillé par rapport au corps inférieur 10, en fin d'utilisation.

On constate que le corps central 1 comporte des découpes formant des rainures et des épaulements qui sont détaillés ci après. Le corps central 1 est fixé au corps inférieur 10 et le manchon actionneur 11 est disposé de manière coulissante à l'intérieur dudit corps inférieur 10.

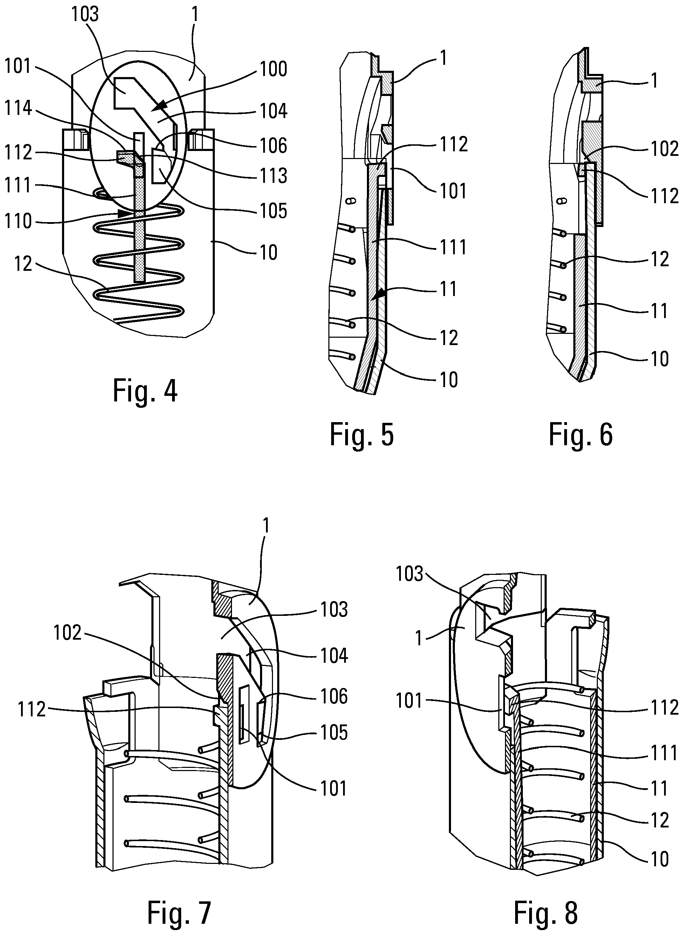

Le corps central 1 comporte une première rainure 101, sensiblement axiale, et une ouverture 103, séparée de ladite première rainure 101 mais disposée dans le prolongement axial de ladite première rainure 101. Ledit corps central 1 comportant également une came radiale 102 disposée entre ladite première rainure 101 et ladite ouverture 103. Comme visible notamment sur les figures 6 et 7, ladite came radiale 102 peut être formée par un épaississement radial incliné de la paroi du corps central 1, ledit épaississement étant formé à l'extrémité axiale de la première rainure 101. Ladite came radiale 102 coopérant avec ladite tête 112 de ladite patte flexible 110 pour déformer radialement ladite patte flexible 110 et ainsi permettre à ladite tête 112 de passer de ladite première rainure 10 à ladite ouverture 103 lors du déplacement du manchon actionneur 11 vers sa position d'actionnement.

Ledit corps central 1 comporte une zone de réception finale 105 décalée axialement et latéralement par rapport à ladite ouverture 103. Comme visible sur les figures, cette zone de réception finale 105 est disposée axialement environ au niveau de ladite première rainure 101. L'ouverture 103 est reliée à ladite zone de réception finale 105 par une rainure inclinée latéralement 104. Un épaulement axial 106 est prévu entre ladite zone de réception finale 105 et ladite rainure inclinée 104. Ainsi, lorsque ledit manchon actionneur 11 revient de sa position d'actionnement vers sa seconde position projetée, ladite tête 112 de la patte flexible 110 coulisse dans ladite rainure inclinée latéralement 104, déformant ainsi latéralement ladite patte flexible 110. Lorsque ledit manchon actionneur 11 atteint sa seconde position projetée, après utilisation, ladite tête 112 vient s'encliqueter sous ledit épaulement axial 106, verrouillant ainsi ledit manchon actionneur 11 par rapport audit corps central 1 et par rapport au corps inférieur 10. A partir de cette position verrouillée, ledit manchon actionneur ne peut plus être déplacé en direction de sa position d'actionnement, de part la butée formée entre la tête 112 de la patte flexible 110 et l'épaulement axial 106.

Les figures 4 à 8 représentent la position de départ, c'est-à-dire au moment où l'utilisateur va commencer à utiliser l'autoinjecteur. On voit sur ces figures que la tête 112 est disposée dans ladite rainure axiale 101 du corps central 1. Lorsque le manchon actionneur 11 coulisse vers l'intérieur du corps inférieur 10, ladite tête 112 de la patte flexible 110 va coulisser à l'intérieur de ladite rainure 101 du corps central. Lorsque la tête 112 atteint l'extrémité axiale de la première rainure 101, ladite came radiale 102 va coopérer avec ladite tête 112. Cette came radiale 102 va donc déformer la patte flexible 110, et notamment sa partie de tige 111, radialement vers l'intérieur en direction de son axe central longitudinal.

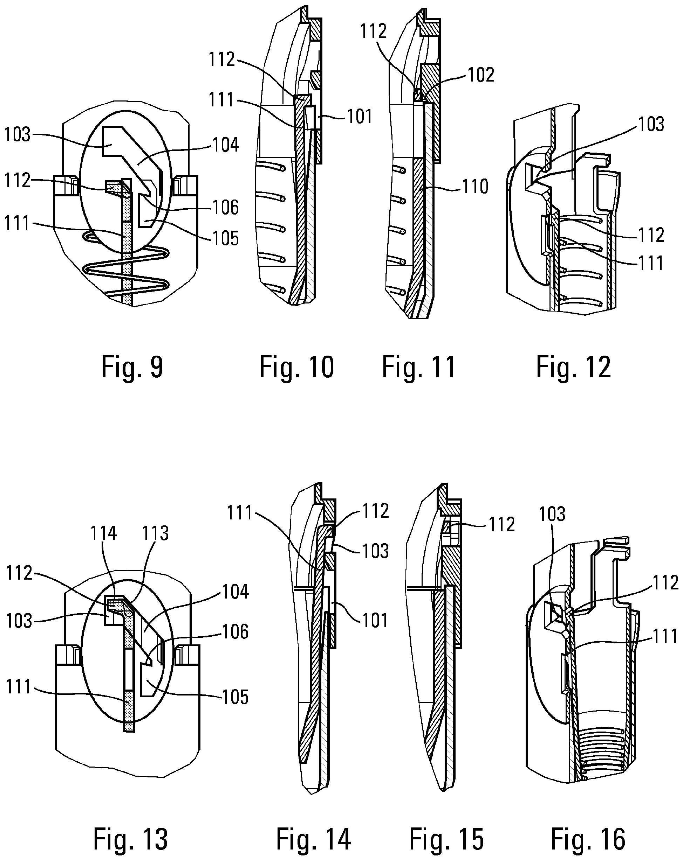

Les figures 9 à 12 illustrent la position dans laquelle la patte flexible 110 est radialement déformée. Comme visible notamment sur la figure 11, après cette déformation radiale, la tête 112 de la patte flexible 110 va continuer à se déplacer axialement sur une distance supplémentaire jusqu'à atteindre ladite ouverture 103. Le manchon actionneur 11 atteint alors sa position d'actionnement, comme représentée sur la figure 13.

Dans cette position d'actionnement, la patte flexible 110 revient élastiquement dans sa position non déformée radialement. La tête 112 de la patte flexible 110 rentre alors à l'intérieur de ladite ouverture 103 comme visible sur la figure 14.

La déformation radiale de la patte flexible 110, nécessaire pour déplacer le manchon actionneur de sa première position projetée vers sa position d'actionnement, génère une certaine résistance. Combinée à la force de compression du ressort 12, cette résistance oblige l'utilisateur à exercer au moins une force prédéterminée pour réaliser le déplacement du manchon actionneur 11 à l'intérieur du corps inférieur 10. Ceci évite tout risque d'actionnement accidentel ou non souhaité après retrait du capot 14. L'actionnement n'a lieu que si l'utilisateur exerce ladite force prédéterminée sur le manchon actionneur 11. Ce seuil de force crée aussi une certaine précompression dans la main de l'utilisateur, avec pour effet que lorsque ce seuil est atteint, le déplacement du manchon actionneur 11 vers sa position d'actionnement est garanti.

Lorsque le manchon actionneur 11 atteint sa position d'actionnement, c'est-à-dire dans la position des figures 13 à 16, le ressort 12 du manchon actionneur a été comprimé et la serrure de piquage est déclenchée par ledit manchon actionneur 11, comme cela sera plus amplement décrit ultérieurement, ce qui provoque le déplacement de la seringue A à l'intérieur du corps inférieur 10 et donc le piquage de l'aiguille dans le corps de l'utilisateur. Pendant toute cette phase de piquage ainsi que pendant la phase d'injection qui suit ladite phase de piquage, le manchon actionneur 11 ne bouge pas par rapport au corps inférieur 10, puisque l'utilisateur maintient sa pression sur la partie du corps dans laquelle il réalise l'injection.

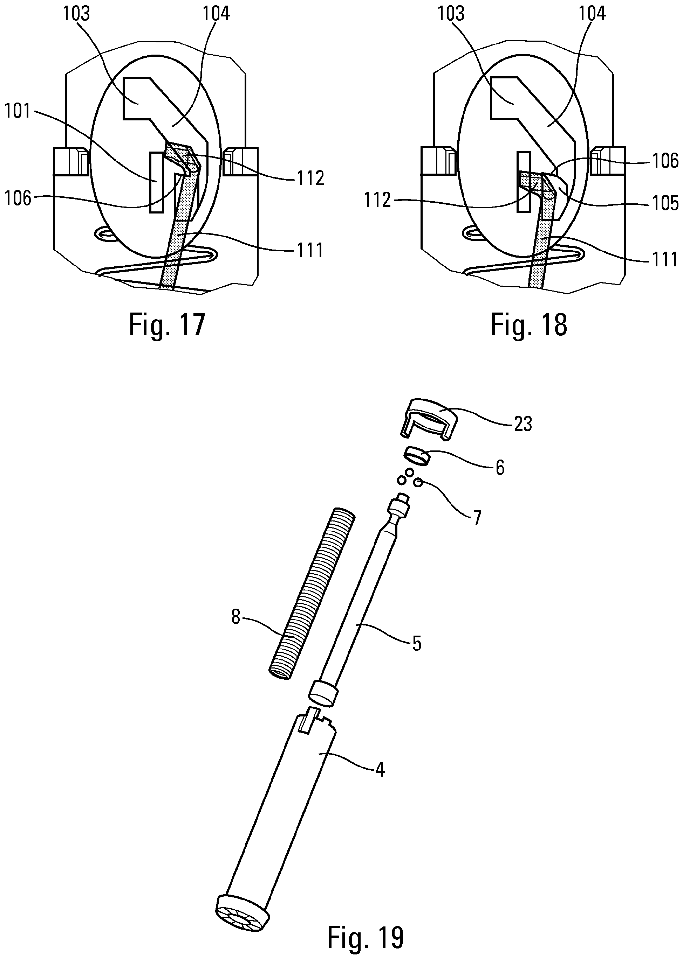

En fin d'utilisation, lorsque l'utilisateur va retirer l'autoinjecteur de son corps, le ressort 12 du manchon actionneur 11 va solliciter ledit manchon actionneur 11 en retour de sa position d'actionnement vers sa seconde position projetée, comme cela est représenté sur la figure 3c. Pendant ce déplacement axial de retour du manchon actionneur 11 dans le corps inférieur 10, la tête 112 de la patte flexible 110 va coopérer avec la rainure inclinée 104 comme visible sur les figures 17 et 18. Ceci va provoquer une déformation élastique de la patte flexible 110, et notamment de sa partie de tige 111, au fur à mesure que le manchon actionneur 11 va coulisser axialement, la tête 112 glissant dans ladite rainure inclinée 104 déformant latéralement ladite patte flexible 110 comme visible clairement sur la figure 17. Cette rainure inclinée 104 se termine dans une zone de réception finale 105 pourvue d'un épaulement axial 106. En fin de course de retour du manchon actionneur 11, la tête 112 de la patte flexible 110 va pénétrer dans cette zone de réception finale 105 et le bord supérieur 114 de la tête 112 va venir coopérer avec l'épaulement axial 106, ce qui va bloquer le manchon actionneur 11 par rapport au corps inférieur 10. Le manchon actionneur 11 ne peut alors plus coulisser axialement vers l'intérieur du corps inférieur 10, et le dispositif de sécurité est alors en position finale verrouillée. Ainsi, l'aiguille est totalement protégée après utilisation et l'utilisateur ne peut plus utiliser l'autoinjecteur ou se blesser avec l'aiguille.

Bien entendu, les formes des rainures, leurs dimensions et leurs inclinaisons peuvent être modifiées en fonction des besoins et des caractéristiques souhaitées pour le dispositif de sécurité aiguille.

Le manchon actionneur décrit ci-dessus est particulièrement efficace et fiable, tout en étant robuste et facile et donc peu coûteux à mouler.

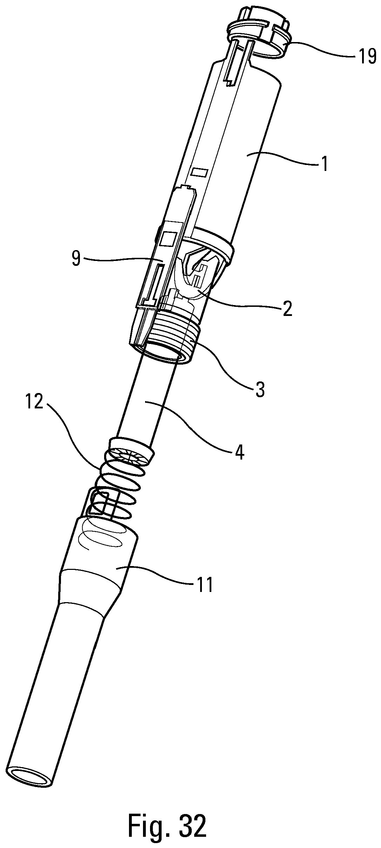

Les figures 32 à 46 décrivent plus particulièrement le dispositif de déplacement de la seringue dans le corps inférieur 10. Ce dispositif de déplacement assure d'une part le piquage, c'est à dire l'insertion de l'aiguille dans le corps de l'utilisateur, et d'autre part la rétractation de l'aiguille après injection.

Comme nous l'avons vu précédemment, en début d'actionnement, la seringue A est déplacée axialement dans ledit corps inférieur 10 pour réaliser l'insertion de l'aiguille dans le corps de l'utilisateur. Après injection du produit fluide dans le corps de l'utilisateur, et éventuellement après un certain retard fourni par le dispositif retardateur décrit ci-dessus, la seringue A est à nouveau déplacée dans l'autre direction à l'intérieur du corps inférieur 10, pour être rétractée et ainsi faire automatiquement sortir l'aiguille du corps de l'utilisateur. De cette manière lorsque l'utilisateur retire l'autoinjecteur de son corps, l'aiguille n'est plus saillante mais au contraire rétractée à l'intérieur dudit autoinjecteur.

Pour réaliser ces déplacements en va-et-vient de la seringue A dans le corps inférieur 10, il est prévu une bague de commande 2 qui coopère avec le manchon de commande 4, avec le coulisseau de commande 9 ainsi qu'avec le manchon actionneur 11. Par ailleurs, le déclencheur 19 intervient pour réaliser la rétractation de la seringue à l'intérieur du corps comme cela sera expliqué ci-après.

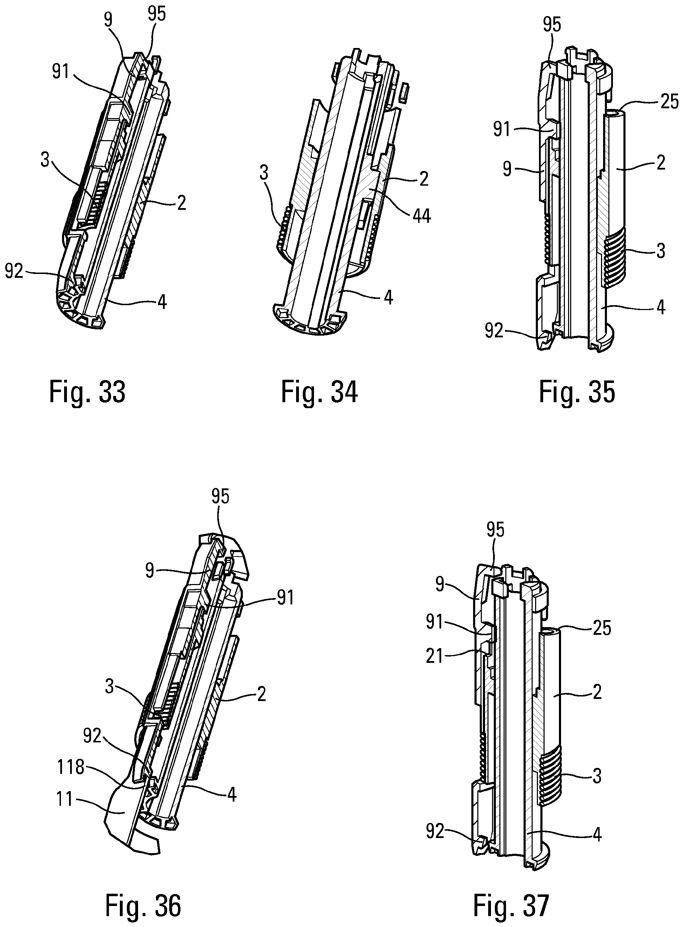

Les figures 33 à 35 illustrent la position de départ avant que la seringue soit déplacée pour le piquage. On constate que la bague de commande 2 est sollicitée en rotation par le ressort de piquage 3, qui ici est un ressort agissant en torsion. Un tel ressort à torsion permet de réaliser un piquage non douloureux.

Dans cette position initiale des figures 33 à 35, la rotation de la bague de commande 2 est empêchée par une projection 91 du coulisseau de commande 9, comme plus clairement visible sur la figure 35.

Lorsque le manchon actionneur 11 arrive dans sa position d'extrémité à l'intérieur du corps inférieur 10, comme représenté sur la figure 3b, un épaulement 118 dudit manchon actionneur 11 va coopérer avec un épaulement 92 du coulisseau de commande 9 pour déplacer axialement ledit coulisseau de commande 9 vers le haut sur la figure 36. Ce déplacement axial du coulisseau de commande 9 va libérer la rotation de la bague de commande 2 qui va pouvoir tourner sous l'effet de son ressort de piquage chargé 3.

La bague de commande 2 comporte trois profils inclinés 24, 25, 26 similaires à des rampes, dont les fonctions seront explicitées ci-après.

La bague de commande 2 comporte un premier profil interne incliné 24, telle qu'une rampe, qui va coopérer avec une projection 44 du manchon de commande 4. Ainsi, la rotation de la bague 2 va progressivement déplacer axialement ledit manchon de commande 4. Ce manchon de commande 4 coopère avec le logement de seringue 13 qui reçoit la seringue, et ainsi un déplacement du manchon de commande déplace la seringue A dans le corps inférieur 10 pour réaliser le piquage de l'aiguille.

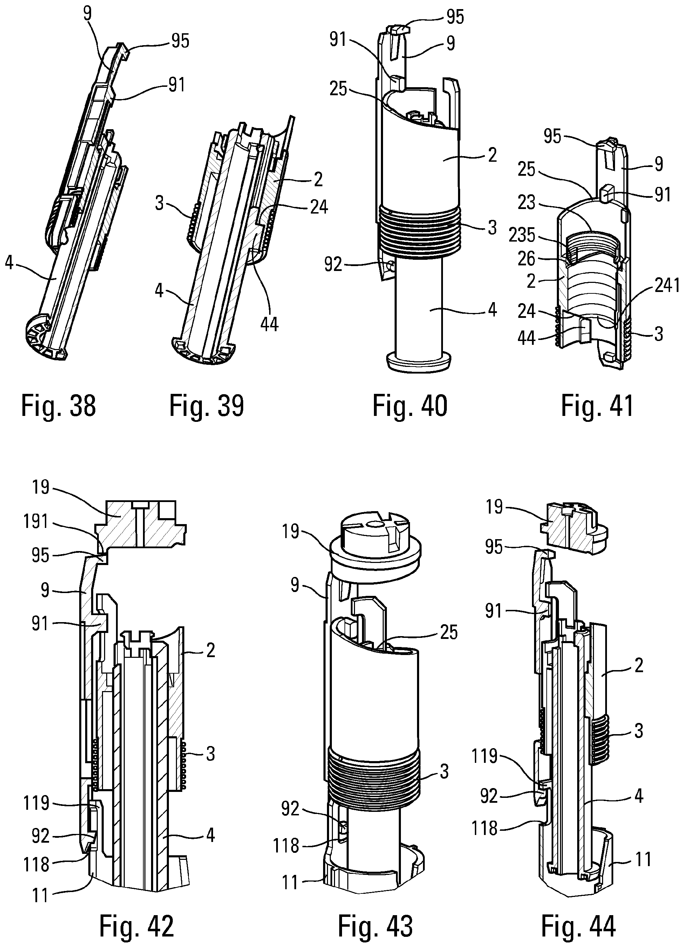

La figure 39 illustre la position dans laquelle l'aiguille est totalement insérée, avec le premier profil incliné 24 qui coopère avec la projection 44 du manchon de commande 4.

Pendant le déplacement du manchon de commande 4 et donc l'insertion de l'aiguille dans le corps de l'utilisateur, la projection 91 du coulisseau de commande est également en contact avec un profil incliné externe 25 de la bague 2, tel qu'une rampe externe, qui va provoquer un déplacement axial supplémentaire dudit coulisseau de commande 9 par rapport au manchon actionneur 11. Ceci va déplacer le coulisseau de commande 9 dans la même direction que le manchon actionneur 11 lors du piquage. De ce fait, la projection 92 du coulisseau de commande 9 vient proche d'une projection supérieure 119 du manchon actionneur 11, et la projection 95 du coulisseau de commande 9 vient proche d'une projection 191 du déclencheur 19, comme visible sur la figure 44.

La première rampe inclinée interne 24 qui coopère avec la projection 44 du manchon de commande 4 comporte avantageusement un plat 241, c'est-à-dire une portion non inclinée, visible sur la figure 41. Ce plat 241 a une fonction très importante puisqu'il garantit que le début de l'injection ne se produira qu'après la fin totale de l'insertion d'aiguille dans le corps de l'utilisateur. Alors que pour beaucoup d'autoinjecteurs il est nécessaire de commencer l'injection un peu avant que l'aiguille atteigne son point final d'insertion, pour des raisons de tolérance de fabrication, le plat 241 sur la rampe 24 permet d'éviter ce phénomène. En effet, alors que la bague 2 a déjà déplacé complètement le manchon de commande 4 axialement et donc a réalisé l'insertion totale de l'aiguille de la seringue dans le corps de l'utilisateur, il est nécessaire que la bague 2 tourne encore sur l'arc de cercle formé par ledit plat, par exemple sur environ 30°, pour déclencher la serrure d'injection. Ainsi, la bague de blocage 23 de la serrure d'injection n'est déplacée hors de sa position de blocage qu'après la rotation supplémentaire de la bague 2 sur l'arc de cercle formé par ledit plat 241. Pendant cette rotation supplémentaire, il n'y a pas de déplacement axial du manchon de commande 4, et donc de la seringue A, puisque le plat 241 n'est pas incliné. Même avec des tolérances de fabrication, on garantit ainsi que l'insertion est terminée avant que l'injection ne commence. Parallèlement, pendant cette rotation supplémentaire de la bague de commande 2, un second profil incliné interne 26, tel qu'une rampe, de la bague de commande 2 va coopérer avec une projection 235 de la bague de blocage 23 de la serrure d'injection et déplacer celle-ci hors de sa position de blocage pour libérer l'injection, lorsque la bague de commande 2 arrive à la fin de sa rotation supplémentaire. Ceci est également visible sur la figure 41. Avantageusement, la bague de commande 2 comporte trois seconds profils inclinés internes 26 disposés à 120° les uns des autres, et la bague de blocage 23 comporte trois projections 235 également disposées à 120° les unes des autres, une projection 235 respective coopérant avec un second profil incliné interne 26 respectif.

Lorsque l'injection est déclenchée par la bague de blocage 23, la rotation de la bague de commande 2 est à nouveau bloquée par le coulisseau de commande 9.

Avec le coulisseau de commande 9 dans la position de la figure 44, si l'utilisateur retire l'autoinjecteur de son corps alors que l'injection est en cours ou après injection mais avant la fin du retardateur, le ressort 12 du manchon actionneur 11 va solliciter ledit manchon actionneur 11 en retour hors du corps inférieur 10. Ce déplacement du manchon actionneur 11 va tirer le coulisseau de commande 9 axialement vers le bas sur la figure 44 par la coopération entre l'épaulement supérieur 119 et la projection 92 du coulisseau. Ainsi, la bague de commande 2 va à nouveau être libérée en rotation par le coulisseau de commande 9, et le ressort 3 va solliciter cette bague de commande davantage en rotation ce qui va provoquer la rétractation de la seringue et de l'aiguille à l'intérieur du corps. Le manchon actionneur 11, en fin de mouvement, sera verrouillé comme décrit précédemment. Ainsi, même si l'utilisateur retire l'autoinjecteur avant la fin de distribution du produit, le dispositif de sécurité aiguille est opérant.

Dans un fonctionnement normal, l'injection se termine et comme cela sera décrit ci-après, la tige de piston 5 va libérer la rotation d'un déclencheur 19, éventuellement avec un certain retard si un dispositif retardateur est utilisé. A partir du moment où le déclencheur 19 a réalisé une rotation prédéfinie, une projection 191 du déclencheur 19 va coopérer avec l'épaulement supérieur 95 du coulisseau de commande 9, et ce coulisseau de commande 9 va être déplacé axialement vers le bas sur la figure 44, ce qui va libérer la rotation de la bague de commande 2 comme décrit précédemment.

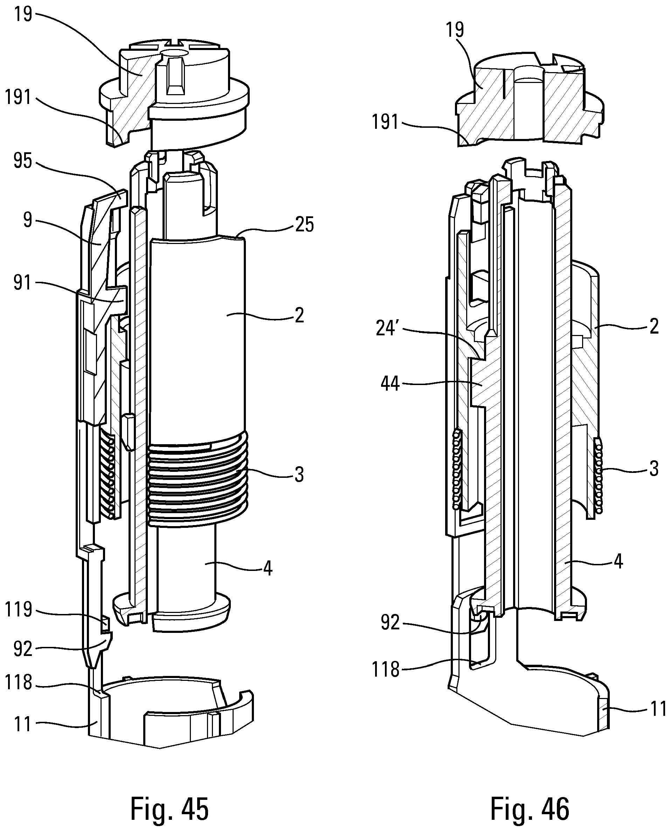

Les figures 45 et 46 illustrent la rétractation de l'aiguille avec la rotation de la bague 2 qui va amener la projection 44 du manchon de commande face à une rainure interne de la bague 2, ce qui provoquera, sous l'effet du ressort, le déplacement axial en retour du manchon de commande 4 à l'intérieur de la bague de commande 2 et donc la rétraction de la seringue et de l'aiguille.

Les figures 19 à 26 illustrent schématiquement une serrure d'injection avantageuse. L'autoinjecteur comporte des moyens d'injection, comprenant notamment la tige de piston 5, le ressort d'injection 8 et la bague de blocage 23, ces moyens d'injection étant bloqués dans une position chargée par ladite serrure d'injection. Le déblocage de ladite serrure d'injection provoque alors l'actionnement desdits moyens d'injection et donc l'injection du produit fluide à travers l'aiguille.

Comme représenté sur la figure 19, ladite serrure d'injection comporte un manchon de commande 4 disposé dans ledit corps central 1, ledit manchon de commande 4 contenant ladite tige de piston 5 et ledit ressort d'injection 8, ladite tige de piston 5 comportant un évidement radial 50 recevant au moins un élément de blocage 7 mobile entre une position de blocage et une position de déblocage. Ledit au moins un élément de blocage 7 est de préférence de forme sensiblement sphérique. Dans la variante représentée, il y a trois éléments de blocage 7 sous forme de billes, mais un nombre différent d'éléments de blocage et des formes un peu différentes de ces éléments de blocage sont envisageables. La description suivante sera néanmoins faite en référence à trois billes, sans que ceci soit limitatif. Lesdites billes 7 sont sollicitées radialement vers l'extérieur par ladite tige de piston 5 et sont retenues en position de blocage par un organe de blocage, qui dans ce mode de réalisation est formé par une bague de blocage 23. Cette bague de blocage 23 est déplaçable axialement par rapport à ladite tige de piston 5 entre une position de verrouillage, dans laquelle elle maintient les éléments de blocage 1007 en position de blocage, et une position de déverrouillage, dans laquelle lesdits éléments de blocage 1007 sont libérés pour ainsi débloquer ladite serrure d'injection, permettant audit ressort d'injection de déplacer ladite tige de piston 5 vers sa position d'injection.

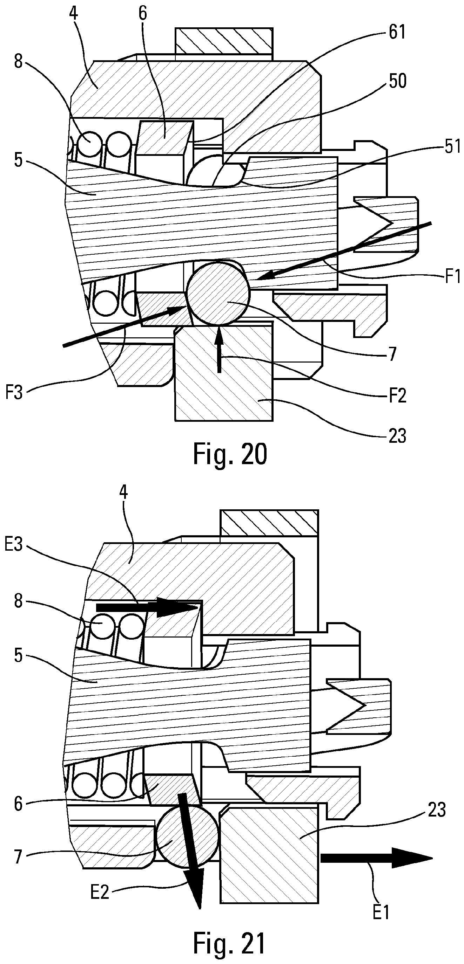

La figure 20 montre la serrure d'injection dans la position de blocage. Le ressort d'injection 8 coopère d'une part avec la tige de piston 5 et d'autre part avec une pastille support 6. Cette pastille support 6 est formée par un anneau disposé autour de ladite tige de piston 5. La tige de piston 5 comporte un évidement périphérique 50, pourvu avantageusement d'une surface inclinée 51, formé par un rétrécissement du diamètre de ladite tige de piston 5. Cette tige de piston 5 est disposée à l'intérieur du corps de commande 4 et est susceptible d'être déplacée axialement vers la gauche sur la figure 20 pour pousser le piston de la seringue A à l'intérieur de la seringue et distribuer le produit fluide contenu dans ladite seringue à travers l'aiguille.

Comme visible sur la figure 20, les billes 7 sont disposées dans ledit évidement 50 formé dans la tige de piston 5 et coopère donc avec d'une part la paroi inclinée 51 de la tige de piston 5 et d'autre part avec la surface supérieure 61 de ladite pastille support 6.

La surface inclinée 51 de la tige de piston est au contact des billes 7 de sorte que sous l'effet du ressort comprimé 8, ladite surface inclinée 51 exerce une force de réaction F1 sur les billes 7, cette force F1 n'étant pas exactement axiale mais dirigée légèrement vers l'extérieur, sollicitant ainsi les billes 7 radialement vers l'extérieur de la position de blocage de la figure 20.

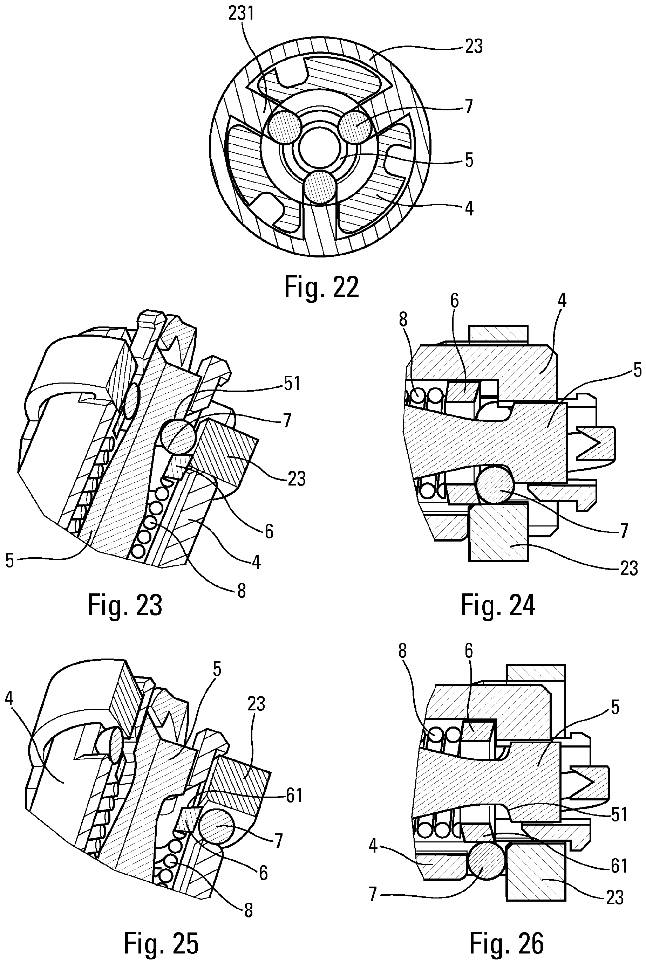

La bague de blocage 23 est prévue radialement à l'extérieur des billes 7 pour bloquer radialement lesdites billes dans la position de blocage. En se référant plus particulièrement à la figure 22, on constate que les billes peuvent être disposées dans des logements du manchon de commande 4, la bague de blocage 23 comportant des projections 231, une pour chaque bille 7, qui viennent se positionner au contact des billes 7 pour éviter que celles-ci soient déplacées radialement vers l'extérieur.

La pastille 6 transmet la force F3 du ressort 8 aux billes 7, et la bague de blocage 23 exerce une force de réaction F2 sur les billes 7 pour empêcher un déplacement radial de celles-ci. Ainsi, ce sont les billes 7 qui supportent tous les efforts exercés sur la serrure dans la position de blocage, avec un équilibre en trois points sous l'effet des forces F1, F2 et F3. Une telle serrure est particulièrement stable et robuste, et permet notamment de résister aux drops tests. Ces tests simulent le fait de laisser tomber l'autoinjecteur par terre après avoir retirer le capot 14, l'objectif étant d'éviter un déclenchement de la serrure d'injection lors de cette chute. En particulier, aucune force n'est exercée sur les pièces de structure de l'autoinjecteur, tel que le corps central 1 ou le corps inférieur 10. Cette serrure permet ainsi d'éviter un risque de démontage intempestif du dispositif pendant un transport ou une manipulation.

Il est à noter que les billes 7 pourraient être remplacées par des éléments non sphériques mais de forme arrondie plus complexe, par exemple en forme de cylindre ou de haricot, afin d'améliorer encore la stabilité de la serrure. Dans ce cas, ces éléments mobiles non sphériques pourraient être réalisés en métal, par exemple par découpe à fil d'acier.

Lorsque l'aiguille de la seringue a complètement pénétré le corps de l'utilisateur, et seulement après cette insertion totale, comme cela sera décrit plus tard, la bague de blocage 23 est déplacée selon la flèche E1 sur la figure 21. Ceci a pour effet de libérer les billes 7 de leur position de blocage, celles-ci étant alors déplacées radialement vers l'extérieur selon la flèche E2. En variante, la bague de blocage 23 pourrait aussi être déplacée en rotation vers une position où elle libère les billes. La pastille support 6 vient alors en butée contre un bord interne du manchon de commande 4 comme représenté par la flèche E3 sur la figure 21. Dans cette position, la tige de piston 5 n'est plus retenue par les billes 7 et elle est donc déplacée axialement, c'est-à-dire vers la gauche sur la figure 21, pour réaliser l'injection du produit. Les billes 7 ne peuvent plus revenir en position de blocage, empêchées par la pastille 6, comme visible sur la figure 21.

Les figures 23 à 26 illustrent avec des vues légèrement différentes les deux positions de blocage et de déblocage de la serrure d'injection comme décrit ci-dessus en référence aux figures 20 et 21.

La serrure d'injection représentée sur les figures 19 à 26 permet de déverrouiller une force importante exercée par un ressort comprimé, en l'occurrence le ressort d'injection 8, en exerçant une force relativement faible et aisément maitrisable sur la bague de blocage 23. En particulier, la force nécessaire pour déplacer ladite bague de blocage 23 en position de déblocage peut représenter seulement 10%, voire même seulement 5%, de la force exercée par le ressort d'injection 8. Ceci représente un rendement très important qui garantit un actionnement aisé et fiable du dispositif.

Lorsque l'injection est terminée, c'est-à-dire lorsque la tige de piston 5 a atteint sa position d'extrémité dans laquelle le piston de la seringue A a été déplacé pour injecter le produit fluide, un déclencheur 19 est actionné pour rétracter la seringue et donc l'aiguille.

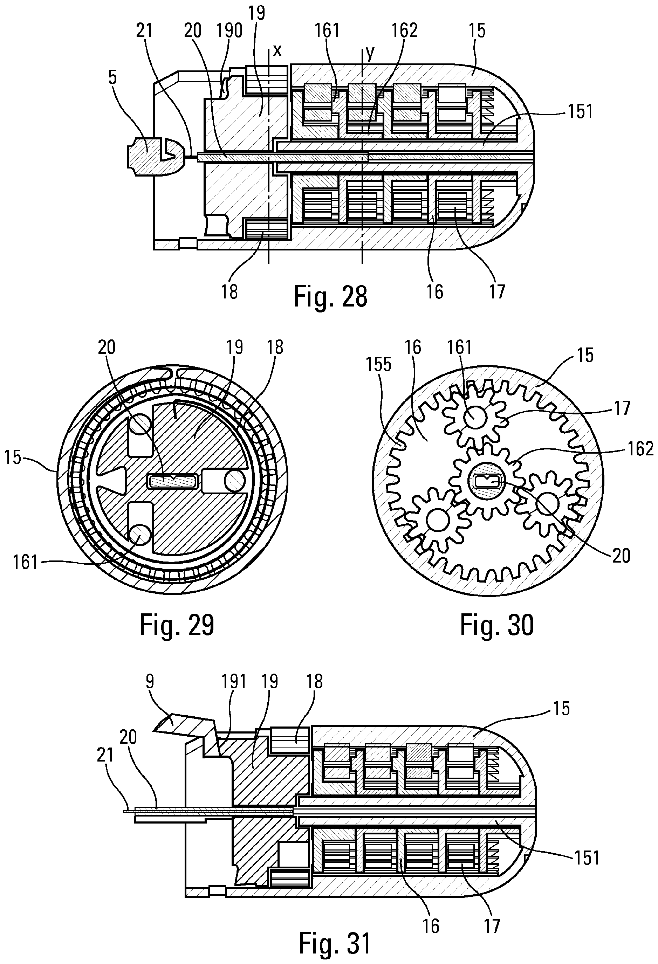

Lors de la phase d'injection, un doigt de verrouillage 20 s'étend à travers le déclencheur 19 et dans le canal central 151 du corps supérieur 15. Un ressort retardateur 18, ici un ressort à spirale, sollicite ledit déclencheur 19 en rotation. Cette rotation est bloquée par le doigt de verrouillage 20, avantageusement de forme oblongue, qui est adapté à tourner ensemble avec ledit déclencheur 19, mais qui est bloqué en rotation par ledit canal central 151 du corps supérieur 15. Pendant la phase d'injection, la tige de piston 5 se déplace axialement, c'est-à-dire vers la gauche sur la figure 28. Au fur à mesure qu'elle se déplace, elle va tirer sur le fil 21 qui va donc s'étendre hors du canal 151. Tant que le doigt de verrouillage 20 est disposé à l'intérieur du canal central 151, la rotation du déclencheur 19 est bloquée. Lorsque la tige de piston 5 approche de la fin de course d'injection, le fil 21 est complètement tiré et tendu entre la tige de piston 5 et le doigt de verrouillage 20, et tout déplacement supplémentaire de la tige de piston 5 va donc déplacer axialement le doigt de verrouillage 20 hors dudit canal central 151. Lorsque la tige de piston 5 atteint position d'extrémité de fin d'injection, le doigt de verrouillage ne coopère plus avec le canal central 151, et le déclencheur 19 et le doigt de verrouillage peut alors tourner sous l'effet du ressort retardateur 18. Comme visible sur la figure 31, le déclencheur 19 comporte une rampe externe 190 inclinée qui peut comporter d'un côté une projection 191. Lorsque le déclencheur 19 aura réalisé une rotation prédéfinie, typiquement environ un tour, cette projection 191 va venir coopérer avec le coulisseau de commande 9, ce qui va déplacer celui-ci axialement et ainsi déclencher la rétractation de l'aiguille, comme cela a été décrit précédemment.

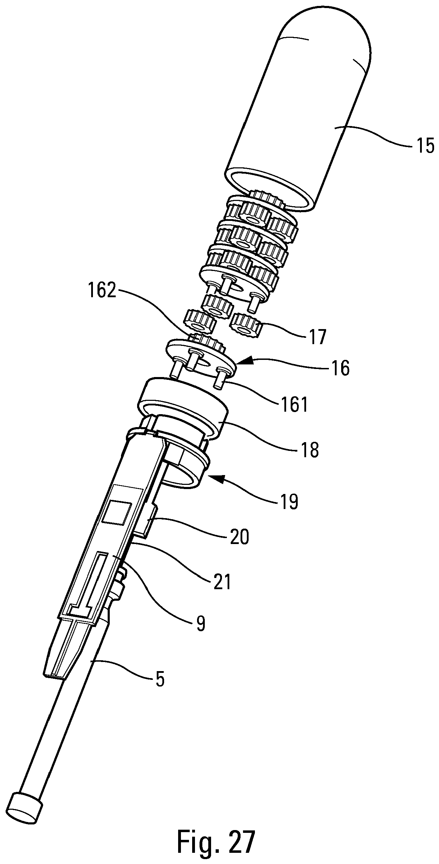

Les figures 27 à 31 illustrent un dispositif retardateur avantageux.

Ce dispositif retardateur, qui est optionnel dans un autoinjecteur, a principalement pour but de décaler dans le temps la rétractation de la seringue A et donc de l'aiguille hors du corps de l'utilisateur après la fin de l'injection du produit fluide à l'intérieur dudit corps. Ceci permet notamment une diffusion pendant quelques secondes du produit après son injection. Un tel retardateur procure aussi un bénéfice pour l'utilisateur, qui n'a plus à compter, par exemple jusqu'à 10, après son injection, le temps pris pour ce comptage pouvant être très variable d'un utilisateur à un autre. Avec un retardateur, on facilite la séquence d'utilisation d'un autoinjecteur.

Le retardateur mécanique représenté sur les figures 27 à 31 permet de décaler ainsi de quelques secondes cette rétractation, ce retard étant prédéterminable.

La figure 27 illustre une vue schématique en éclatée de ce dispositif retardateur. Celui-ci comprend le corps supérieur 15, plusieurs planétaires 16 avec plusieurs satellites 17, le ressort retardateur 18, le déclencheur 19, le doigt de verrouillage 20, le fil 21 et la tige de piston 5. C'est cette tige de piston 5 qui va réaliser l'actionnement du dispositif retardateur lorsqu'elle arrive en fin de course d'injection avec la totalité du produit qui a été injecté.

La figure 28 montre le dispositif retardateur avant son actionnement. On voit que la tige d'actionnement 5 est connectée au doigt de verrouillage 20 par l'intermédiaire du fil 21. Dans cette position, le fil 21 et le doigt de verrouillage 20 s'étendent à l'intérieur d'un canal central 151 du corps supérieur 15 et dans le déclencheur 19. Le corps supérieur 15 comporte un engrenage 155 sur sa surface interne latérale, comme clairement visible sur la figure 30. Cet engrenage interne 155 du corps supérieur 15 coopère avec une pluralité de satellites 17 qui sont assemblés sur des planétaires 16. Dans l'exemple représenté sur la figure 28, il y plusieurs planétaires empilés axialement l'un sur l'autre. Les planétaires 16 comportent une plaque en forme de disque sur laquelle sont formées d'un côté des tiges de support de satellites 161 qui reçoivent chacune de manière rotative un satellite 17. Dans l'exemple représenté, il y a trois satellites 17 à chaque étage de sorte qu'il y a trois tiges 161. Chaque planétaire 16 associé à ses satellites 17 forme un étage du dispositif retardateur. De l'autre côté de la plaque en forme de disque, le planétaire 16 comporte un engrenage 162 adapté à coopérer avec les satellites 17 de l'étage adjacent. Ainsi, comme visible sur la figure 30, le dispositif retardateur utilise le principe des trains épicycloïdaux. Chaque étage de ce dispositif permet de démultiplier et/ou ralentir les rotations de l'étage précédent.

Lorsqu'un dispositif retardateur est utilisé, le déclencheur 19 coopère avec un premier planétaire 16, dont les tiges 161 s'étendent à l'intérieur dudit déclencheur 19. L'engrenage 162 de ce premier planétaire 16 coopère alors avec les satellites d'un second planétaire adjacent, qui coopèrent avec l'engrenage latéral 155 du corps supérieur 15, démultipliant ainsi la rotation du premier planétaire et donc du déclencheur, et donc freinant cette rotation. Chaque étage supplémentaire du train épicycloïdal formant le retardateur va davantage démultiplier ces rotations, et donc davantage freiner la rotation du déclencheur 19. Ainsi, avec quatre étages comme représenté sur les figures, on peut ramener à un seul tour la rotation du déclencheur 19 alors que le dernier planétaire 16 disposé tout au fond du corps supérieur 15 réalisera simultanément environ cinquante tours.

Selon le nombre d'étages et/ou selon de nombre de satellites et/ou selon la forme des planétaires et/ou selon les dimensions des engrenages en jeu, on peut régler assez précisément le retard entre le moment où le dispositif retardateur est déclenché et le moment où le déclencheur 19 aura réalisé sa rotation prédéfinie pour déclencher la rétractation de la seringue, comme cela sera explique ultérieurement. Un freinage par frottements peut aussi être prévu, par exemple entre les satellites 17 et l'engrenage interne 155 du corps supérieur 15.

Le dispositif retardateur permet donc de décaler d'un temps prédéterminé le moment où ledit déclencheur va actionner la rétractation de l'aiguille, à partir du moment où la phase d'injection est terminée.

Il est à noter que le principe du fil déployable connecté d'une part à la tige de piston 5 et d'autre part au doigt de verrouillage 20, peut être utilisé sans le système de train épicycloïdal tel que représenté sur les figures 27 à 31, comme cela sera notamment décrit ci-après en référence au second mode de réalisation. Ce fil, très peu encombrant, permet de garantir que la rétractation de l'aiguille ne commence qu'une fois la phase d'injection totalement terminée, en permettant notamment de compenser d'éventuelles tolérances de fabrication. Plus généralement, l'utilisation d'un fil permet de réduire l'encombrement du dispositif. De ce fait, on peut l'utiliser avantageusement pour différentes fonctions dans un autoinjecteur, dès lors qu'on a besoin de tirer une pièce par rapport à une autre.

Selon un aspect avantageux, la coque externe 22 comporte plusieurs indicateurs qui permettent d'informer l'utilisateur de l'avancement des séquences de piquage, d'injection et de rétractation. En cas d'utilisation d'un dispositif retardateur, on peut aussi prévoir un affichage dudit retard.

Ainsi, comme visible sur les figures 2a à 2f, la coque externe 22 peut comporter plusieurs fenêtres de visualisation, en l'occurrence trois fenêtres 221, 222, 223, qui permettent de visualiser des éléments mobiles lors des différentes phases d'actionnement, ces éléments comportant des indicateurs, typiquement des couleurs.

Ainsi, le coulisseau de commande 9, qui au repos est dans une première position par rapport au corps central 1, se déplace axialement vers une seconde position lors du déplacement du manchon actionneur 11. Il reste ensuite dans cette seconde position pendant toute la phase d'injection, et revient en direction de sa première position lors de la rétractation de l'aiguille. Ce n'est que lorsque le manchon actionneur retourne à sa seconde position projetée, que le coulisseau de commande atteint cette première position. Ce coulisseau de commande 9 peut comporter un ou plusieurs indicateurs de couleur, par exemple une zone rouge corne visible sur la figure 1. Ce coulisseau peut donc être utilisé pour indiquer d'une part la position projetée du manchon actionneur 11 (première position) et d'autre part la phase de piquage et d'injection (seconde position).

Le déclencheur 19, qui déclenche la rétractation de l'aiguille en fin d'injection, peut aussi comporter un indicateur, par exemple une zone rouge, qui s'affiche lorsque ledit déclencheur a réalisé sa rotation prédéfinie et ainsi actionné la rétractation de l'aiguille.

Ainsi, la première fenêtre de visualisation 221 peut être la fenêtre de fin d'injection, c'est-à-dire que lorsqu'une couleur prédéfinie, par exemple le rouge, apparaît dans la fenêtre 221, l'injection est terminée et la seringue a été rétractée. L'utilisateur sait donc que lorsque cette première fenêtre de visualisation est rouge, il peut retirer l'autoinjecteur en toute sécurité de son corps. Cette indication peut par exemple être fournie par le déclencheur 19.

La seconde fenêtre de visualisation 222 peut être celle des phases de piquage et d'injection, qui passe au rouge pendant du début de la phase de piquage à la fin de la phase d'injection. Ceci permet d'éviter que l'utilisateur ne retire l'autoinjecteur de son corps pendant ces phases, qui peuvent durer plusieurs secondes. Cette indication peut être fournie par le coulisseau actionneur 9.

La troisième fenêtre de visualisation 223 peut être celle du manchon actionneur 11, avec le rouge affiché lorsque le manchon actionneur 11 est dans une position projetée hors du corps inférieur. Cette troisième fenêtre de visualisation 223 est donc rouge avant actionnement, puis à nouveau après utilisation, lorsque le manchon actionneur 11 est verrouillé en position de sécurité. Cette indication peut être fournie par le coulisseau de commande 9. Dans l'exemple représenté, la zone rouge du coulisseau actionneur 9 passe de la troisième fenêtre de visualisation 223, avant actionnement (figure 2a) à la seconde fenêtre de visualisation 222 (figure 2c) lorsque le manchon actionneur est en position d'actionnement où il déclenche la phase de piquage. Pendant cette transition, ladite zone rouge n'est pas visible, étant située entre lesdites fenêtres 223 et 222 (figure 2b). Pendant les phases de piquage et d'injection, le coulisseau de commande reste dans sa seconde position (figure 2c & figure 2d). Lorsque le coulisseau de commande 9 est à nouveau déplacé axialement vers sa première position par le déclencheur 19, pour actionner la rétractation de l'aiguille, la zone rouge repasse entre la seconde fenêtre 222 à la troisième fenêtre 223, étant à nouveau invisible (figure 2e), pour finalement réapparaitre dans la troisième fenêtre 223 lorsque le manchon actionneur est verrouillé en seconde position projetée (figure 2f).

Dans cette configuration, la combinaison du rouge dans les première et troisième fenêtres de visualisation 221 et 223 garantit la fin du processus d'utilisation de l'autoinjecteur, avec l'aiguille rétractée et le manchon actionneur 11 verrouillé, ce qui garantit une sécurité optimale.

Bien entendu, d'autres moyens d'affichage ou d'indication sont aussi possibles, et ladite coque externe 22 peut comporter un nombre quelconque de fenêtre de visualisation, de formes et de dimensions quelconques, et qui pourraient être positionnées différemment de la variante représentée sur les figures. Une même fenêtre peut notamment afficher plusieurs fonctions différentes.

Eventuellement, dans la première fenêtre de visualisation 221 ou dans une autre fenêtre de visualisation, par exemple une fenêtre de visualisation supplémentaire, on peut prévoir d'afficher l'état du dispositif retardateur, par exemple avec un décompte. Celui-ci pourrait être réalisé par exemple avec des valeurs numériques inscrites sur le bord latéral externe du déclencheur qui passe progressivement dans une fenêtre de visualisation appropriée et qui affiche en seconde le décompte du retardateur. D'autres variantes sont bien entendu également possibles.

Cette coque externe 22 peut aussi comporter un ou des boutons de piquage et/ou de rétractation de l'aiguille, si l'autoinjecteur prévoit de tels boutons pour réaliser le piquage et/ou la rétractation de l'aiguille.

La coque externe 22 pourrait aussi comporter un indicateur de température du produit à injecter. En effet, beaucoup de produits à injecter sont stockés à 8° et il est souvent recommandé de les sortir 30-60 min a l'avance. Si le produit est trop froid au moment de l'injection, ceci peut provoquer des douleurs chez le patient. Par exemple, la coque 22 pourrait comporter un affichage de la température du réservoir contenant le produit à injecter. En variante, on pourrait aussi prévoir une étiquette qui change de couleur avec la température. Cet indicateur de température pourrait être prévu sur la coque, ou sur le réservoir, notamment la seringue, et être visible à travers une fenêtre de la coque.

Les figures 47 à 74c illustrent plusieurs variantes d'un second mode de réalisation de l'invention. Ce second mode de réalisation concerne un autoinjecteur simplifié, constitué de moins de pièces, et donc plus simple et moins coûteux à fabriquer et à assembler.

Dans la variante de la figure 47 de ce second mode de réalisation, l'autoinjecteur comporte un corps inférieur 1010, un manchon actionneur 1011, un ressort 1012 du manchon actionneur, un capot 1014, un manchon de commande 1004, une tige de piston 1005, une pastille support 1006, trois éléments de blocage 1007, ici sous forme de billes, un ressort d'injection 1008, un organe à clic 1500, un fil 1021, et une coque externe 1022.

Le capot 1014 permet notamment de verrouiller l'autoinjecteur pendant le transport et le stockage. Tant que ce capot est assemblé sur le corps inférieur 1010, il empêche tout actionnement du manchon actionneur 1011, et donc tout déclenchement de l'autoinjecteur.

Comme pour le premier mode de réalisation, la seringue A est une seringue pré-remplie. Elle comporte avantageusement un capuchon d'aiguille B qui protège et isole l'aiguille avant utilisation de l'autoinjecteur. Avantageusement, ce capuchon d'aiguille B est retiré automatiquement au moment du retrait du capot 1014 à partir du corps inférieur 1010.

Il est à noter que ce second mode de réalisation présente plusieurs éléments similaires au premier mode de réalisation, ces éléments similaires étant désignés par des références numériques similaires à celles du premier mode de réalisation, augmentées de 1000. Ainsi, par exemple, le manchon actionneur référencé 11 dans le premier mode de réalisation est maintenant référencé 1011. Par conséquent, dans la description de ce second mode de réalisation, ce sont principalement les différences par rapport au premier mode de réalisation qui seront décrites, étant entendu que les autres éléments et fonctions demeurent similaires sinon identiques entre les deux modes de réalisation.

La principale différence de ce second mode de réalisation est que le réservoir, en l'occurrence la seringue A, est fixe par rapport au corps inférieur 1010, par rapport au manchon de commande 1004 et par rapport à la coque externe 1022. Ainsi, pour réaliser le piquage de l'aiguille, seul le manchon actionneur coulisse par rapport au reste de l'autoinjecteur. Il n'y a donc pas dans ce second mode de réalisation de dispositif de déplacement de seringue.

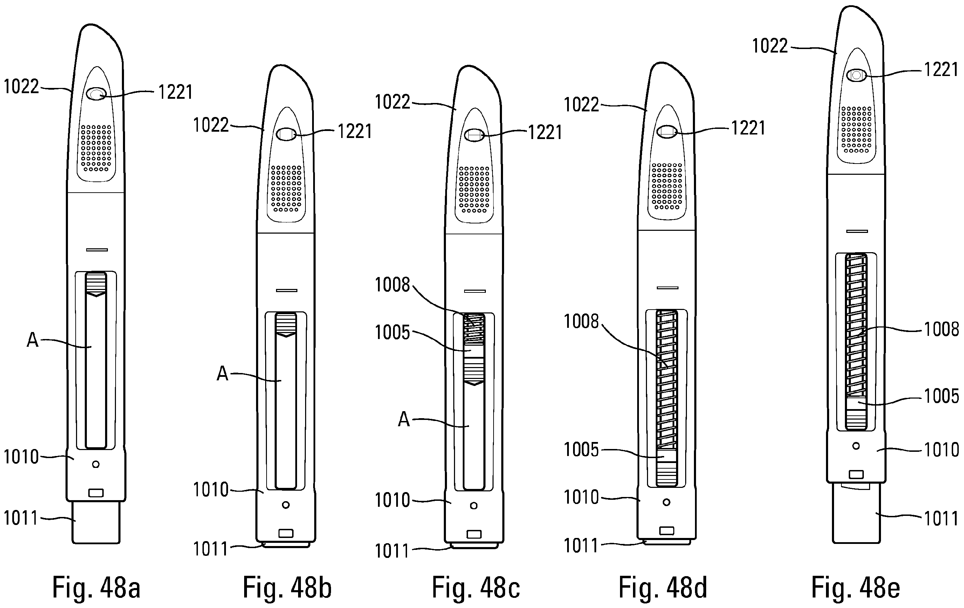

Les figures 48a à 48e illustrent les séquences de l'utilisation de l'autoinjecteur de la figure 47.

Sur la figure 48a, l'autoinjecteur est en position de repos avant utilisation, le capot 1014 ayant été retiré.

Lorsque l'utilisateur veut utiliser l'autoinjecteur, il prend le dispositif, par exemple au niveau de la coque externe 1022 et il appuie le manchon actionneur 1011, qui dans une première position projetée fait saillie hors du corps inférieur 1010, contre la partie du corps où il veut réaliser l'injection. Sur la figure 48b, on voit que la pression exercée par l'utilisateur sur le manchon actionneur 1011 provoque le coulissement de celui-ci vers l'intérieur du corps inférieur 1010, ce qui découvre l'aiguille et donc son piquage en raison de la pression exercée par l'utilisateur sur l'autoinjecteur.

Lorsque le manchon actionneur 1011 atteint sa position d'actionnement, qui est sa position d'extrémité à l'intérieur du corps inférieur 1010, il provoque le déclenchement de la phase d'injection, ce qui est représenté sur les figures 48c et 48d. On constate que la tige de piston 1005 coulisse à l'intérieur de la seringue A en poussant le piston de celle-ci sous l'effet du ressort d'injection 1008. Le produit est donc distribué.

A la fin de l'injection, lorsque l'utilisateur retire l'autoinjecteur du site d'injection, le manchon actionneur 1011 est à nouveau déplacé hors du corps inférieur 1010 vers une seconde position projetée, sous l'effet du ressort du manchon actionneur, avec un verrouillage dudit manchon actionneur 1011, ce qui garantit une sécurité absolue pour l'utilisateur et évite tout risque de blessures avec l'aiguille après utilisation du dispositif. Il est à noter que les première et seconde positions projetées du manchon actionneur, qui, dans l'exemple représenté, sont des positions différentes, pourraient éventuellement être identiques.

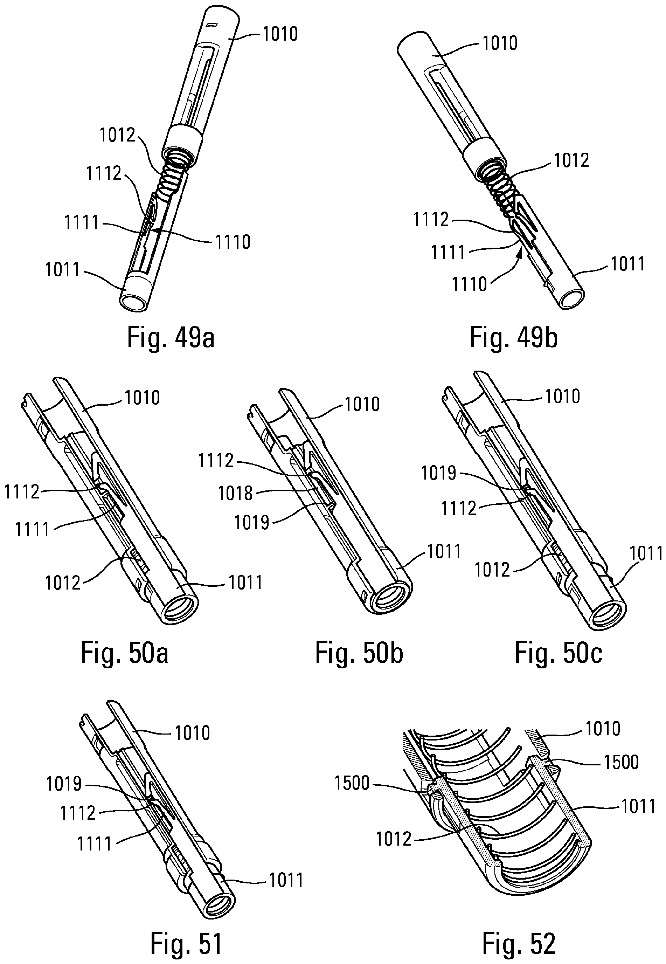

Dans ce second mode de réalisation, comme visible notamment sur les figures 49a à 52, ledit manchon actionneur 1011 comporte également une patte flexible 1110 qui n'est flexible que latéralement, c'est-à-dire qu'elle ne se déforme que dans la direction périphérique du manchon actionneur 1011. Un manchon actionneur 1011 pourvu d'une telle patte flexible est encore plus simple à mouler que la patte flexible à double flexibilité du premier mode de réalisation, ce qui est favorable du point de vue des coûts de fabrication. Avec une patte flexible seulement latéralement, on gagne aussi en encombrement radial, ce qui permet notamment d'améliorer l'esthétique de l'autoinjecteur. La patte flexible 1110 comporte avantageusement une partie de tige 1111 qui est flexible et qui se termine par une partie de tête 1112.

Dans une première variante, illustrée sur la figure 51, ladite patte flexible 1110 est adaptée à se déformer latéralement par rapport audit corps inférieur 1010 d'une part lorsque ledit manchon actionneur 1011 est déplacé de sa première position projetée vers sa position d'actionnement et d'autre part lorsque ledit manchon actionneur 1011 est déplacé de sa position d'actionnement en retour vers sa seconde position projetée. Dans ce cas, la tête 1112 de la patte flexible doit surmonter une résistance pour se déformer latéralement au début de l'actionnement, pour créer une sorte de précompression qui garantit que lorsque le manchon actionneur va coulisser vers l'intérieur du corps inférieur 1010, l'aiguille va à tous les coups pénétrer le site d'injection jusqu'à sa position d'injection souhaitée. Dans l'exemple de la figure 51, cette résistance est formée par un épaulement 1019 du corps inférieur 1010.

De préférence toutefois, dans une seconde variante représentée sur les figures 50a, b, c et 52, ladite patte flexible 1110 n'est pas déformée lorsque ledit manchon actionneur 1011 se déplace de sa première position projetée, avant actionnement, vers sa position d'actionnement, et ladite patte flexible est déformée latéralement seulement lorsque ledit manchon actionneur 1011 se déplace de sa position d'actionnement vers sa seconde position projetée, en fin d'utilisation. Dans cette variante, avant actionnement, le manchon actionneur 1011 est relié audit corps inférieur 1010 par au moins un pont sécable 1500. Ce mode de réalisation permet notamment un moulage aisé, et donc des coûts de fabrication réduits, une adaptation et une gestion de l'effort de rupture des ponts sécables facilités par le dimensionnement de ces ponts, et une fonction de témoin d'utilisation.

La figure 52 illustre deux ponts sécables 1500, adaptés à se rompre et donc à permettre un coulissement du manchon actionneur 1011 par rapport au corps inférieur 1010 lorsque l'utilisateur appuie l'autoinjecteur sur le site d'injection avec une pression prédéterminée.

Lorsque le manchon actionneur 1011 revient de sa seconde position d'actionnement vers sa position projetée, sous l'effet du ressort 1012, lorsque l'utilisateur retire l'autoinjecteur du site d'injection, le fonctionnement de la patte flexible 1110 peut être identique à celui décrit dans le cadre du premier mode de réalisation, avec une rainure inclinée, une zone de réception finale et un épaulement axial coopérant avec la tête de la patte flexible pour la bloquer en seconde position projetée.

Dans une variante, le corps inférieur 1010 peut comporter un épaulement 1019 qui se prolonge axialement vers l'intérieur par une rampe 1018, par exemple formée par une nervure, qui est au moins partiellement inclinée. Ainsi, lorsque le manchon actionneur 1011 revient de sa position d'actionnement vers sa seconde position projetée, la tête 1112 de la patte flexible 1110 va être déformée latéralement par ladite rampe 1018, pour finalement revenir s'encliqueter sous la projection 1019 en seconde position projetée pour bloquer le manchon actionneur.

Dans la variante de la figure 51, cette même projection 1019 peut coopérer avec la tête 1112 de la patte flexible 1110 à la fois au début d'actionnement pour créer la précompression et en fin d'actionnement pour bloquer le manchon actionneur en seconde position projetée. Bien entendu, on peut aussi prévoir deux épaulements différents pour réaliser ces deux fonctions.

Il est à noter que la patte flexible 1110 peut être fixée audit manchon actionneur 1011 seulement au niveau de sa partie de tige 1111, avec dans ce cas la tête 1112 formant une extrémité libre de ladite patte flexible. En variante, la patte flexible pourrait aussi être fixée audit manchon actionneur des deux cotés, avec ainsi la tête 1112 disposée entre les deux points de fixation. Cette mise en oeuvre renforce notamment la robustesse de la patte flexible. Cette variante pourrait aussi être adaptée à la patte flexible du premier mode de réalisation.

Dans les variantes des figures 49a à 52, la patte flexible 1110 du manchon actionneur 1011 coopère avantageusement avec une ouverture 103, une rainure inclinée 104, une zone de réception finale 105 et un épaulement axial 106 du corps 1010 qui sont similaires à ces mêmes éléments décrits précédemment en référence aux figures 4 à 18.

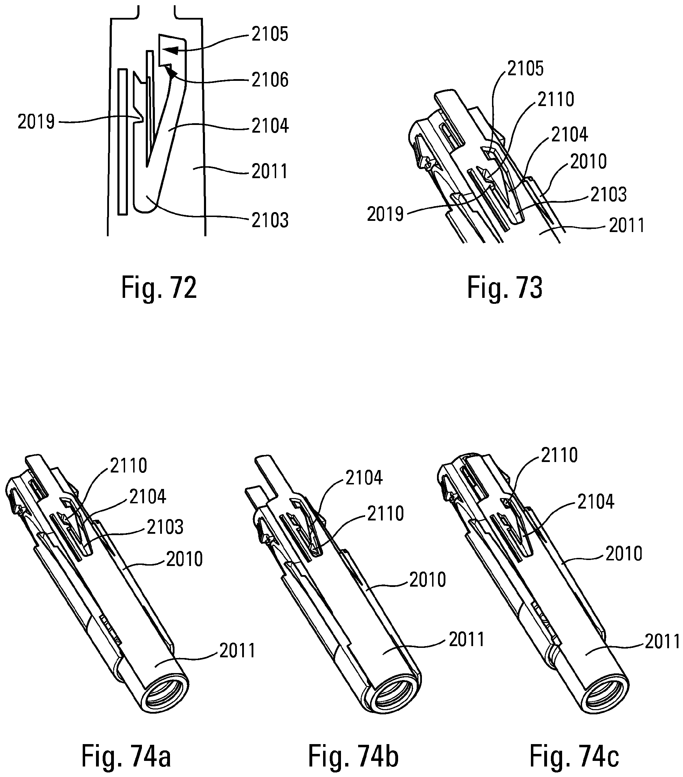

Les figures 72 à 74c illustrent une autre variante de réalisation du manchon actionneur. Dans cette variante, les références numériques seront similaires à celles ci-dessus, mais augmentées de 1000. Ainsi par exemple, le manchon actionneur sera référencé 2011. Dans cette variante particulière, les fonctions du manchon actionneur 2011 et du corps 2010 sont inversées, le corps 2010 comportant la patte flexible 2110, et le manchon actionneur 2011 comportant le profil qui va coopérer avec ladite patte flexible 2110. Le fonctionnement reste toutefois similaire à celui décrit précédemment, avec la patte flexible 2110 qui va progressivement glisser dans ledit profil, et notamment dans une rainure inclinée 2104 qui relie une ouverture 2103 à une zone de réception finale 2105. Pour verrouiller le dispositif en fin d'actionnement dans la zone de réception finale 2105, la patte flexible 2110 va s'encliqueter sur l'épaulement 2106, comme visible sur la figure 74c. Comme décrit précédemment, la patte flexible doit avantageusement surmonter une résistance pour se déformer au début de l'actionnement, pour créer une sorte de précompression qui garantit que lorsque le manchon actionneur 2011 va coulisser vers l'intérieur du corps inférieur 2010, l'aiguille va à tous les coups pénétrer le site d'injection jusqu'à sa position d'injection souhaitée. Dans l'exemple des figures 72 à 74C, cette résistance est formée par un épaulement 2019 du manchon actionneur 2011. Il est à noter que la patte flexible 2110 peut être formée de manière monobloc sur le corps 2010, ou en variante être formée sur une pièce séparée assemblée sur ledit corps 2010, par exemple pour des raisons de simplification et/ou de moulage.

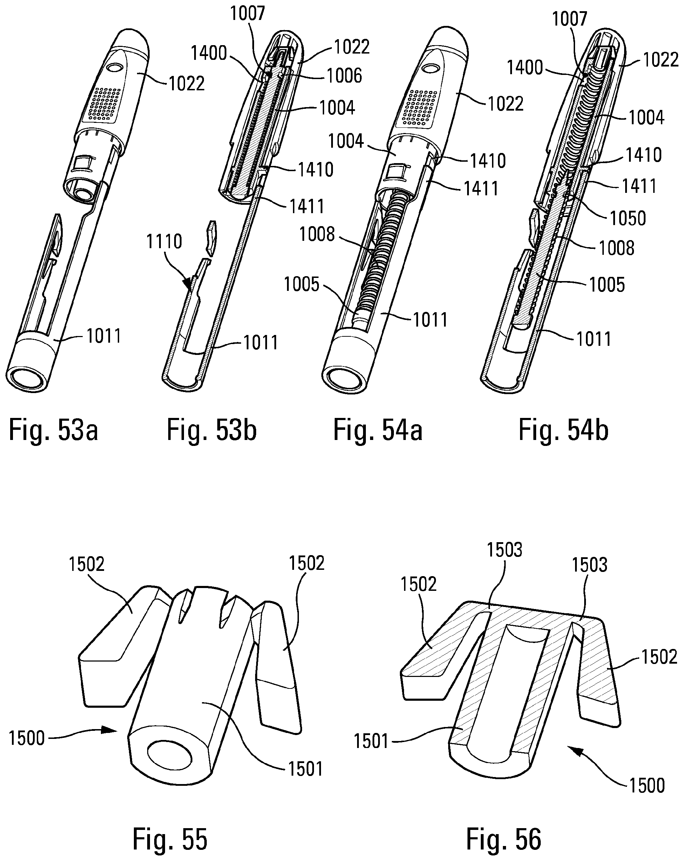

Les figures 53a, b, 54a, b, 57a, b, c et 58a, b, c illustrent l'adaptation au second mode de réalisation de la serrure d'injection décrite dans le premier mode de réalisation.

Comme décrit précédemment, l'autoinjecteur comporte des moyens d'injection, comprenant notamment la tige de piston 1005 et le ressort d'injection 1008, ces moyens d'injection étant bloqués dans une position chargée par ladite serrure d'injection. Le déblocage de ladite serrure d'injection provoque alors l'actionnement desdits moyens d'injection et donc l'injection du produit fluide à travers l'aiguille.

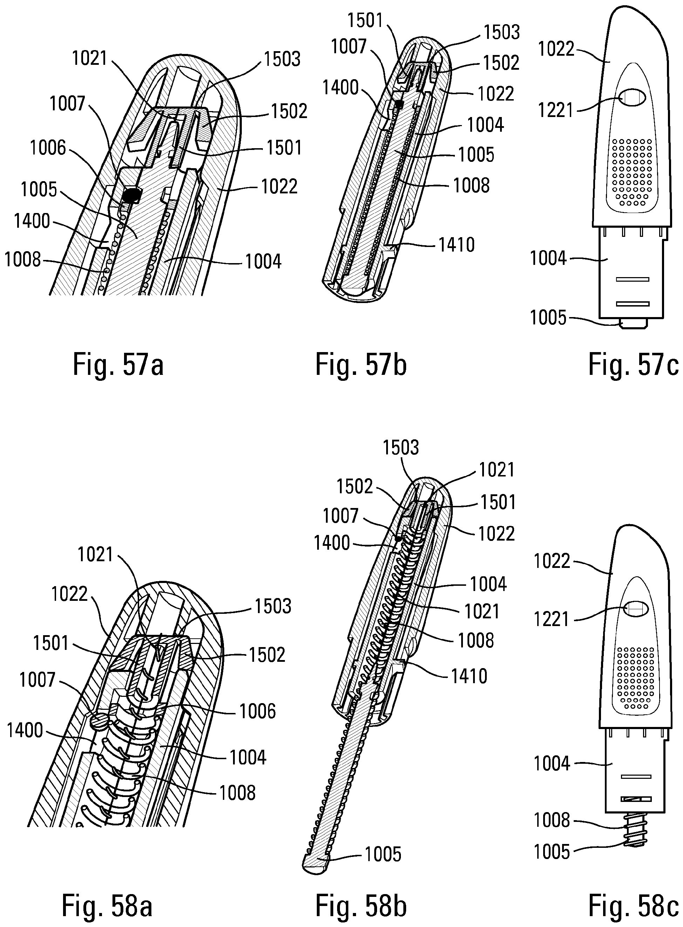

Comme représenté sur les différentes figure 53, 54, 57 et 58 ladite serrure d'injection comporte un manchon de commande 1004 disposé dans ladite coque externe 1022, ledit manchon de commande 1004 contenant ladite tige de piston 1005, ledit ressort d'injection 1008 et une pastille support 1006. Dans la position de blocage représentée sur les différentes figures 53 et 57, le ressort d'injection 1008 coopère d'une part avec la tige de piston 1005 et d'autre part avec ladite pastille support 1006. Cette pastille support 1006 est formée par un anneau disposé autour de ladite tige de piston 1005. Ladite tige de piston 1005 comportant au moins un évidement radial 1050 recevant au moins un élément de blocage 1007 mobile entre une position de blocage et une position de déblocage. Avantageusement, il y a trois éléments de blocage 1007, de préférence de forme sensiblement sphérique, notamment sous forme de billes, mais un nombre différent d'éléments de blocage et des formes arrondies différentes de ces éléments de blocage sont envisageables. Lesdits éléments de blocage 1007 sont sollicitées radialement vers l'extérieur par ladite tige de piston 1005 et sont retenues en position de blocage par un organe de blocage, qui dans ce second mode de réalisation est formé par ledit manchon de commande 1004. Ce manchon de commande 1004 est déplaçable axialement par rapport à ladite tige de piston 1005 entre une position de verrouillage, dans laquelle il maintient les éléments de blocage 1007 en position de blocage, et une position de déverrouillage, dans laquelle lesdits éléments de blocage 1007 sont libérés pour ainsi débloquer ladite serrure d'injection, permettant audit ressort d'injection de déplacer ladite tige de piston 1005 vers sa position d'injection.

Comme visible plus particulièrement sur les figures 57a et 58a, le manchon de commande 1004 comporte une ou plusieurs fenêtres 1400 qui permettent le passage des éléments de blocage 1007 lorsque le manchon de commande a été déplacé vers sa position de déverrouillage, représentée notamment sur les figures 58. Le déplacement du manchon de commande 1004 de sa position de verrouillage vers sa position de déverrouillage est réalisé par une projection 1411 du manchon actionneur 1011, qui va coopérer avec un épaulement 1410 du manchon de commande 1004, de telle sorte que le manchon de commande 1004 est en position de déverrouillage lorsque le manchon actionneur 1011 est en position d'actionnement. Ladite tige de piston 1005 n'étant alors plus bloquée par lesdits éléments de blocage 1007, elle est déplacée hors du manchon de commande par ledit ressort d'injection 1008 précomprimé pour déplacer le piston dans le réservoir et ainsi injecter le produit à travers l'aiguille. Ce type de serrure permet un déblocage a faible effort garantissant ainsi un confort sonore et tactile à l'utilisateur lors de l'injection.

Avantageusement, l'autoinjecteur comporte un dispositif d'indication sonore et/ou tactile 1500 pour indiquer par un son audible ou par une indication tactile à l'utilisateur que la phase d'injection est terminée. Ce dispositif sera décrit ci-après en relation avec trois variantes du second mode de réalisation, mais il pourrait aussi être adapté à un autoinjecteur réalisé selon le premier mode de réalisation.

Selon une première variante de réalisation, ce dispositif d'indication sonore et/ou tactile 1500 comporte une pièce centrale 1501 pourvue d'au moins une pièce latérale 1502 reliée à ladite pièce centrale 1501 par une liaison pliable et/ou sécable 1503. Dans l'exemple représenté sur les figures 55 à 59c, il y a deux pièces latérales 1502 reliée chacune à la pièce centrale par une liaison sécable.

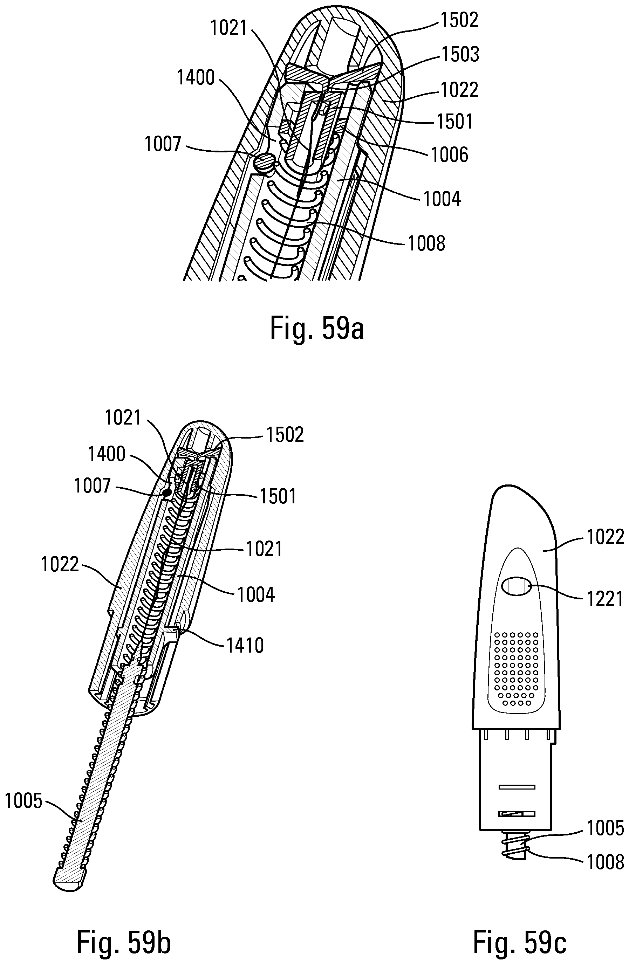

La pièce centrale 1501 est reliée à ladite tige de piston 1005 par ledit fil 1021, qui est fixé d'une part sur ladite pièce centrale 1501 et d'autre part sur ladite tige de piston 1005. En position de blocage de la serrure d'injection, avant le début de l'injection, le fil 1021 est enroulé autour de la tige de piston et la pièce centrale 1501 est disposée à l'extérieur du manchon de commande 1004. Lorsque le manchon de commande 1004 est déplacé vers sa position de déverrouillage, représentée notamment sur la figure 58a, un bord supérieur dudit manchon de commande 1004 vient en contact avec lesdites pièces latérales 1502. Lors de l'injection, lorsque la tige de piston 1005 se déplace par rapport au manchon de commande 1004, le fil 1021 va progressivement se dérouler jusqu'à être tendu en fin d'injection comme représenté sur la figure 58b. A partir de ce moment là, le fil 1021 va exercer une traction sur la pièce centrale 1501, provoquant sous l'effet de la réaction du bord supérieur du manchon de commande 1004, le déplacement et/ou la déformation des pièces latérales. Dans l'exemple représenté avec des liaisons 1503 sécables, celles-ci se cassent, permettant aux pièces latérales 1502 de se déplacer au dessus de la pièce centrale 1501, et donc au manchon de commande 1004 de se déplacer axialement par rapport à la coque externe 1022, comme visible notamment sur la figure 59a. Ce déplacement se faisant sous la pression exercée par le ressort d'injection 1008 sur le manchon de commande 1004, il est relativement brusque et crée un choc entre le manchon de commande 1004, les pièces latérales 1502 et/ou la coque externe 1022. Ce choc est audible et/ou tactile pour l'utilisateur, qui reçoit donc une information sur la fin d'injection. Après actionnement de ce dispositif d'indication sonore et/ou tactile, le fil 1021 n'est plus tout à fait tendu, comme illustré schématiquement sur les figures 59a et 59b.

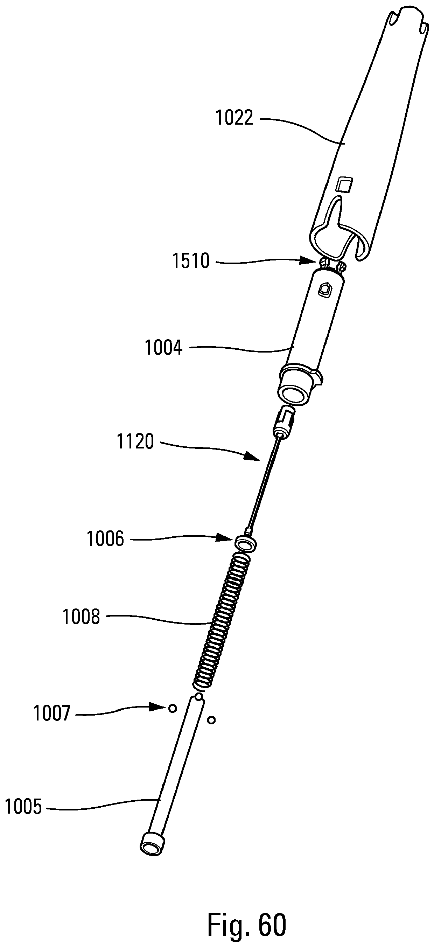

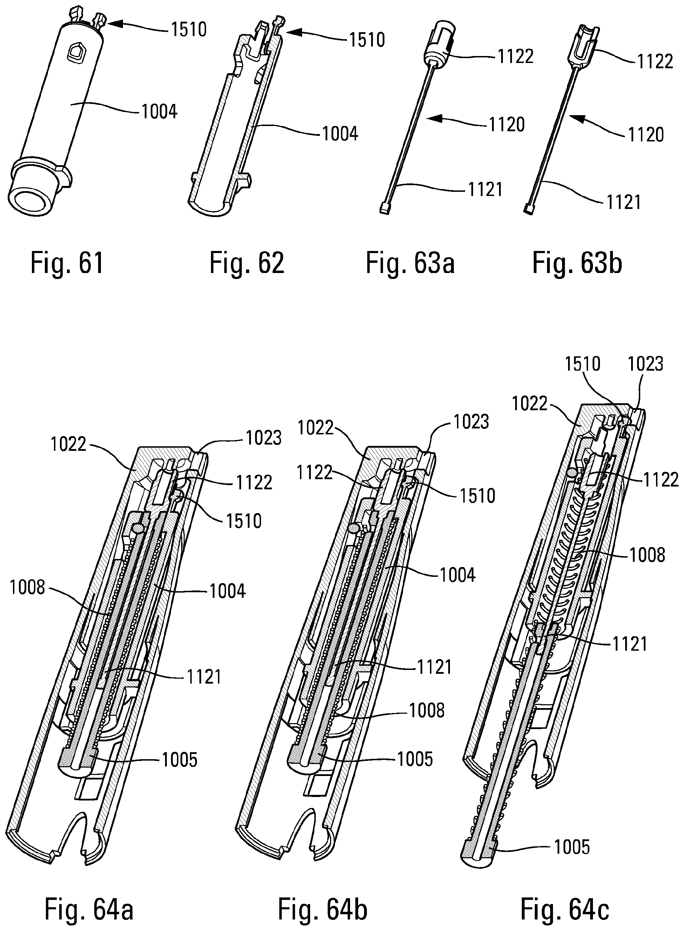

Les figures 60 à 64c illustrent une seconde variante du dispositif d'indication sonore et/ou tactile. Dans cette seconde variante, la pièce centrale est supprimée. Le dispositif d'indication sonore et/ou tactile 1500 comporte un élément mobile qui est ici formé par le manchon de commande 1004, qui comporte à son extrémité distale par rapport à l'aiguille, une ou plusieurs pattes déformables 1510 qui en fin d'injection vont venir buter contre la coque externe 1022. Ce manchon de commande 1004 est dans une première position par rapport à la coque externe 1022 avant actionnement de l'autoinjecteur, comme représenté sur la figure 64a. Lors de l'actionnement, l'ouverture de la serrure d'injection, et donc le début de la phase d'injection, provoque le déplacement du manchon de commande 1004 vers une seconde position, visible sur la figure 64B. Une pièce centrale appelée clé 1120 remplace ici avantageusement le fil de la première variante précédente. Cette clé 1120, notamment visible sur les figures 63a et 63b, comporte une partie de tige 1121 qui s'étend à l'intérieur de la tige de piston 1005, cette partie de tige étant similaire au fil de la première variante. La clé 1120 comporte aussi une partie de tête 1122, disposée à l'extrémité supérieure (ou distale par rapport à l'aiguille) de ladite clé. Cette partie de tête coopère avec lesdites pattes déformables 1510 du manchon de commande 1004, pour les empêcher de se déformer radialement vers l'intérieur. De ce fait, ces pattes déformables 1510 bloquent ledit manchon de commande dans sa seconde position par rapport à ladite coque externe 1022. L'extrémité inférieure (ou proximale par rapport à l'aiguille) de la partie de tige 1121 coopère avec la tige de piston 1005 en toute fin d'injection, provoquant un coulissement de ladite clé 1120 par rapport au manchon de commande 1004 et à la coque externe 1022. Ainsi, après ce coulissement, la partie de tête 1122 ne coopère plus avec les pattes 1510 du manchon de commande, qui peuvent alors de déformer radialement vers l'intérieur. Ceci a pour effet de débloquer le manchon de commande 1004, qui est alors déplacé vers une troisième position contre ladite coque externe 1022 sous l'effet de la force exercée par le ressort d'injection 1008. Ceci crée un choc audible ou autrement détectable par l'utilisateur, qui sait alors que l'injection est terminée.

Avantageusement, la coque externe 1022 comporte une ou plusieurs, notamment trois, fenêtres de visualisation 1023 dans lesquelles lesdites pattes déformables 1510 deviennent visibles en même temps qu'elles viennent taper contre la coque externe. Ceci permet une indication visuelle simultanément à l'indication sonore et/ou tactile.

Avantageusement, ladite au moins une fenêtre de visualisation 1023 est formée sur ou d ans le bord d'extrémité distal de ladite coque externe 1022, en étant visible à la fois dans la direction axiale et dans la direction radiale de ladite coque. Cette mise en oeuvre évite de masquer la ou les fenêtres de visualisation 1023 lorsque l'autoinjecteur est pris en main par l'utilisateur, ce qui garantit une bonne visualisation des informations affichées dans ladite au moins une fenêtre de visualisation 1023 pendant toute la phase d'utilisation, du début jusqu'à la fin. Avec plusieurs fenêtres de visualisation 1023, notamment trois, réparties autour du bord d'extrémité distale du corps 1022, on garantit une parfaite visualisation quelque soit l'orientation de l'autoinjecteur au moment de son utilisation.

Les variantes de réalisation du second mode de réalisation de l'autoinjecteur décrit ci-dessus définissent donc un manchon de commande 1004 ayant trois positions distinctes: avant injection quand il est en position de verrouillage, pendant injection quand il est en position de déverrouillage, et après injection quand il a actionné le dispositif d'indication sonore et/ou tactile. Ceci permet de visualiser aisément ces trois positions distinctes dans une fenêtre de visualisation appropriée 1221. Bien entendu, la coque externe 1022 de ce second mode de réalisation pourrait en outre aussi comporter plusieurs fenêtres de visualisation comme décrit dans le premier mode de réalisation.

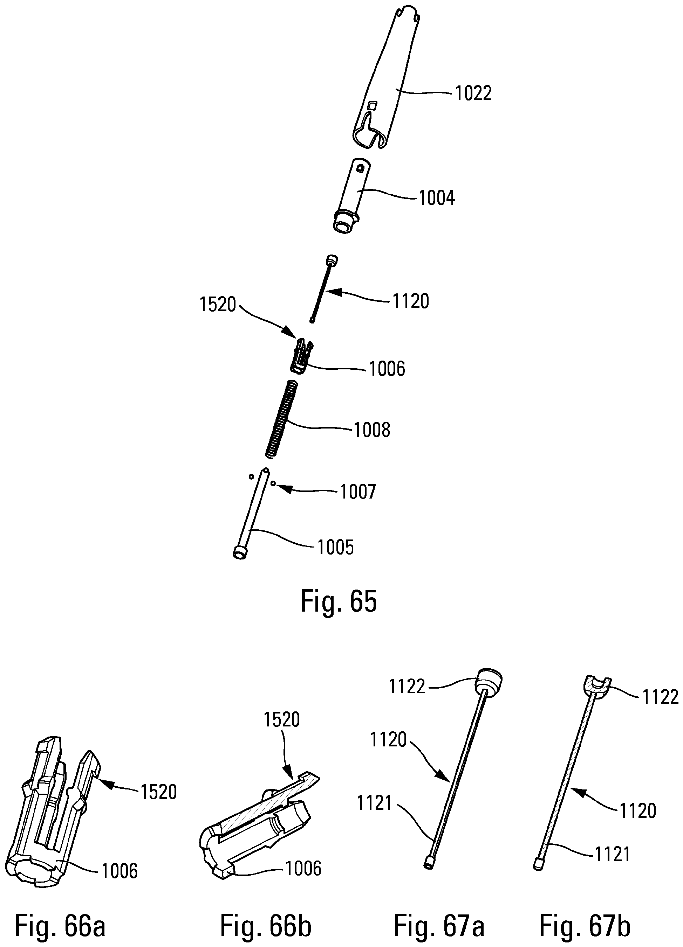

Les figures 65 à 71 illustrent une troisième variante de réalisation du dispositif d'indication sonore et/ou tactile. Dans cette variante, qui est similaire à la seconde variante ci-dessus, l'élément mobile du dispositif d'indication sonore et/ou tactile n'est pas formé par le manchon de commande 1004 mais par la pastille de support 1006 sur laquelle s'appuie le ressort d'injection 1008. Les figures 66a et 66b montre cette pastille de support, qui comporte une ou plusieurs pattes déformables 1520, qui sont similaires aux pattes déformables 1510 du manchon de commande 1004 de la seconde variante ci-dessus. Le fonctionnement est aussi similaire, avec la clé 1120 qui bloque par sa tête 1122 la déformation radiale desdites pattes 1520, ce qui bloque la pastille support par rapport à la coque externe. Lorsque la tige de piston fait coulisser la clé 1120 en fin d'injection, par traction sur la partie de tige 1121, la partie de tête 1122 de la clé 1120 va libérer lesdites pattes 1520, qui, en se déformant radialement vers l'intérieur, vont permettre à ladite pastille support 1006 d'être projetée contre ladite coque externe, créant une indication sonore et/ou tactile. Avantageusement, comme décrit précédemment, une indication visuelle est aussi fournie par une ou des fenêtres de visualisation 1023 de la coque externe qui montrent les pattes déformable 1520 de la pastille support 1006.

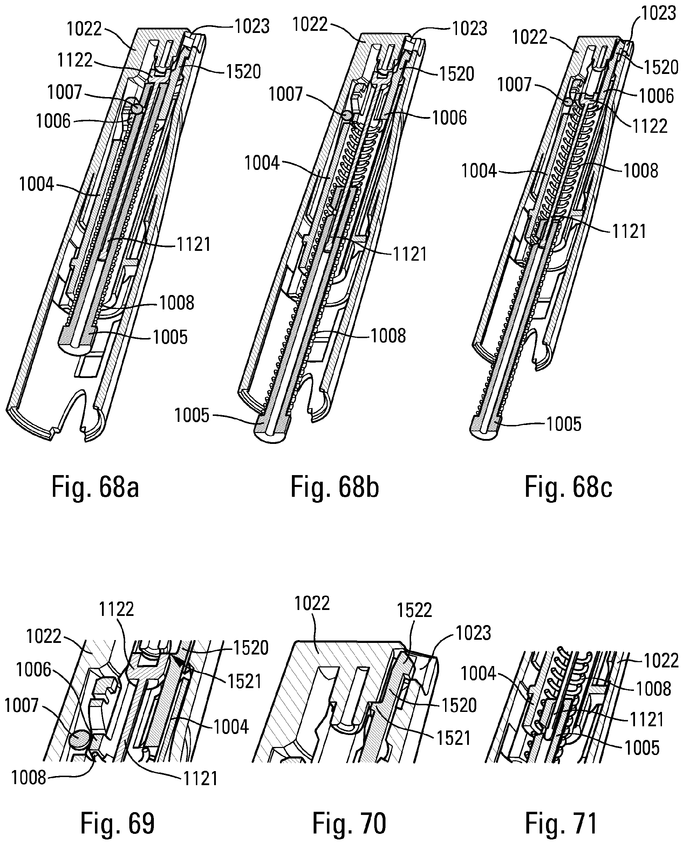

Les figures 69 à 71 illustrent plus en détail le fonctionnement du dispositif d'indication sonore et/ou tactile. Sur la figure 69, la patte déformable 1520 est empêchée de se déformer radialement vers l'intérieur par la présence de la partie de tête 1122 de la clé 1120. Sur la figure 70, la clé a été déplacée par la tige de piston, et par conséquent, la patte déformable 1520 s'est déformée radialement vers l'intérieur. Ceci a provoqué le déplacement de la pastille de support 1006 dans la coque externe, avec un épaulement 1521 de la patte déformable qui vient buter sur une partie de ladite coque externe, générant l'indication sonore et/ou tactile, par exemple un son audible ou une vibration sensible. Simultanément, l'extrémité de la patte déformable 1520 s'est positionnée dans la fenêtre 1023 de la coque externe 1022, comme visible sur la figure 70. La figure 71 illustre la fin de l'injection, avec la tige de piston 1005 qui va tirer sur la partie de tige 1121 de la clé 1120 pour déplacer celle-ci.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à plusieurs modes et variantes de réalisation avantageux, qui combinent plusieurs modules fonctionnels, il est entendu que les différents modules décrits peuvent être utilisés indépendamment les uns des autres. En particulier, le manchon actionneur et/ou le dispositif de déplacement de seringue pour le piquage et/ou la rétractation et/ou la serrure d'injection et/ou le dispositif retardateur et/ou le dispositif d'indication sonore et/ou tactile pourrai(en)t être utilisé(s) indépendamment les uns des autres. Le piquage de l'aiguille et/ou la rétractation de l'aiguille après injection pourrait être commandé par un ou plusieurs bouton(s). Le dispositif d'indication sonore et/ou tactile du second mode de réalisation pourrait être utilisé avec un autoinjecteur du type décrit dans le premier mode de réalisation. D'autres modifications sont également possibles pour l'homme du métier sans sortir du cadre de la présente invention tel que défini par les revendications annexées.

## Revendications

1. Autoinjecteur comportant un corps inférieur (10 ; 1010) et un manchon actionneur (11; 1011), ledit corps inférieur (10; 1010) recevant un réservoir, ledit réservoir contenant du produit fluide et comportant un piston et une aiguille, tel qu'une seringue pré-remplie (A), ledit manchon actionneur (11; 1011) étant pourvu d'une extrémité de contact destinée à venir en contact avec le corps de l'utilisateur, ledit manchon actionneur (11; 1011) étant déplaçable dans ledit corps inférieur (10; 1010) entre des positions projetées, dans lesquelles ledit manchon actionneur (11; 1011) fait au moins partiellement saillie hors dudit corps inférieur (10; 1010) et une position d'actionnement, dans laquelle ledit manchon actionneur (11; 1011) est axialement déplacé vers l'intérieur dudit corps inférieur (10; 1010), ledit autoinjecteur comportant une tige de piston (5; 1005) adaptée à coopérer avec le piston dudit réservoir, ladite tige de piston (5; 1005) étant déplaçable entre une position chargée et une position d'injection dans laquelle ladite tige de piston (5; 1005) a déplacé le piston du réservoir pour injecter le produit fluide à travers l'aiguille, un ressort d'injection (8; 1008) étant prévu pour solliciter ladite tige de piston (5; 1005) vers sa position d'injection, l'autoinjecteur comportant une serrure d'injection qui bloque ladite tige de piston (5; 1005) dans sa position chargée, ladite serrure d'injection comportant un manchon de commande (4; 1004), ledit manchon de commande (4; 1004) contenant ladite tige de piston (5; 1005) et ledit ressort d'injection (8; 1008), ladite tige de piston (5; 1005) comportant un évidement radial (50; 1050) recevant au moins un élément de blocage sphérique (7; 1007) mobile entre une position de blocage et une position de déblocage, ledit au moins un élément de blocage (7; 1007) étant sollicité radialement vers l'extérieur par ladite tige de piston (5; 1005) et étant retenu en position de blocage par une organe de blocage (23; 1004), ladite organe de blocage (23; 1004) étant déplaçable par rapport à ladite tige de piston (5; 1005) pour libérer ledit au moins un élément de blocage (7; 1007) et ainsi débloquer ladite serrure d'injection, permettant audit ressort d'injection de déplacer ladite tige de piston (5; 1005) vers sa position d'injection, ledit manchon actionneur (11; 1011), en position d'actionnement, provoquant le déplacement dudit organe de blocage (23; 1004) de sa position de verrouillage vers sa position de déverrouillage, pour déclencher ladite serrure d'injection, **caractérisé en ce que** ledit au moins un élément de blocage sphérique (7 ; 1007) est une bille.

2. Autoinjecteur selon la revendication 1, dans lequel ledit ressort d'injection (8; 1008) coopère avec une pastille support (6; 1006), sollicitée axialement contre ledit au moins un élément de blocage (7; 1007) en position de blocage, de sorte que toutes les forces (F1, F2, F3) existantes dans la serrure d'injection en position de blocage sont supportées par ledit au moins un élément de blocage (7; 1007).

3. Autoinjecteur selon la revendication 2, dans lequel ladite pastille support (6; 1006) comporte un anneau disposé autour de ladite tige de piston (5; 1005).

4. Autoinjecteur selon l'une quelconque des revendications précédentes, dans lequel ledit évidemment (50; 1050) de ladite tige de piston (5; 1005) comporte une surface inclinée (51) sollicitant ledit au moins un élément de blocage (7; 1007) radialement vers l'extérieur.

5. Autoinjecteur selon l'une quelconque des revendications précédentes, dans lequel la force nécessaire pour déplacer ledit organe de blocage (23; 1004) de sa position de verrouillage vers sa position de déverrouillage représente moins de 10%, avantageusement environ 5%, de la force exercée par ledit ressort d'injection (8; 1008) sur ladite serrure d'injection en position de blocage.

6. Autoinjecteur selon l'une quelconque des revendications précédentes, dans lequel ledit organe de blocage est une bague de blocage (23) déplaçable axialement par rapport au manchon de commande (4), une bague de commande (2) coopérant avec ledit manchon actionneur (11) étant prévu pour coopérer avec ladite bague de blocage (23) pour la déplacer de sa position de verrouillage vers sa position de déverrouillage.

7. Autoinjecteur selon l'une quelconque des revendications 1 à 5, dans lequel ledit organe de blocage est formé par ledit manchon de commande (1004), ledit manchon actionneur (1011) coopérant directement avec ledit manchon de commande (1004) pour le déplacer de sa position de verrouillage vers sa position de déverrouillage.

## Patentansprüche

1. Autoinjektor, aufweisend einen unteren Körper (10; 1010) und eine Betätigungsmuffe (11; 1011), wobei der untere Körper (10; 1010) einen Behälter aufnimmt, wobei der Behälter ein fluides Produkt enthält und einen Kolben und eine Nadel aufweist, wie eine vorgefüllte Spritze (A), wobei die Betätigungsmuffe (11; 1011) mit einem Kontaktende versehen ist, das dazu gedacht ist, mit dem Körper des Benutzers in Kontakt zu kommen, wobei die Betätigungsmuffe (11; 1011) in dem unteren Körper (10; 1010) zwischen ausgefahrenen Positionen, in denen die Betätigungsmuffe (11; 1011) zumindest teilweise aus dem unteren Körper (10; 1010) hervorsteht, und einer Betätigungsposition verschiebbar ist, in der die Betätigungsmuffe (11; 1011) axial zum Inneren des unteren Körpers (10; 1010) verschoben wird, wobei der Autoinjektor eine Kolbenstange (5; 1005) aufweist, die dazu geeignet ist, mit dem Kolben des Behälters zusammenzuwirken, wobei die Kolbenstange (5; 1005) zwischen einer belasteten Position und einer Injektionsposition verschiebbar ist, in der die Kolbenstange (5; 1005) den Kolben des Behälters verschoben hat, um das fluide Produkt durch die Nadel zu injizieren, wobei eine Injektionsfeder (8; 1008) vorgesehen ist, um die Kolbenstange (5; 1005) in ihre Injektionsposition vorzuspannen, wobei der Autoinjektor einen Injektionsverschluss aufweist, der die Kolbenstange (5; 1005) in ihrer belasteten Position sperrt, wobei der Injektionsverschluss eine Steuermuffe (4; 1004) aufweist, wobei die Steuermuffe (4; 1004) die Kolbenstange (5; 1005) und die Injektionsfeder (8; 1008) enthält, wobei die Kolbenstange (5; 1005) eine radiale Vertiefung (50; 1050) aufweist, die mindestens ein kugelförmiges Sperrelement (7; 1007) aufnimmt, das zwischen einer Sperrposition und einer Entsperrposition beweglich ist, wobei das mindestens eine Sperrelement (7; 1007) durch die Kolbenstange (5; 1005) radial zum Inneren gespannt ist und von einem Sperrorgan (23; 1004) in der Sperrposition gehalten wird, wobei das Sperrorgan (23; 1004) in Bezug auf die Kolbenstange (5; 1005) zum Freisetzen des mindestens einen Sperrelements (7; 1007) und dadurch Entsperren des Injektionsverschlusses verschiebbar ist, wodurch ermöglicht wird, dass die Injektionsfeder die Kolbenstange (5; 1005) in ihre Injektionsposition verschiebt, wobei die Betätigungsmuffe (11; 1011) in der Betätigungsposition die Verschiebung des Sperrorgans (23; 1004) aus seiner Verriegelungsposition in seine Entriegelungsposition bewirkt, um den Injektionsverschluss auszulösen, **dadurch gekennzeichnet, dass** das mindestens eine kugelförmige Sperrelement (7; 1007) eine Kugel ist.

2. Autoinjektor nach Anspruch 1, wobei die Injektionsfeder (8; 1008) mit einem Trägerplättchen (6; 1006) zusammenwirkt, das in der Sperrposition axial gegen das mindestens eine Sperrelement (7; 1007) gespannt ist, so dass die gesamten, in der Sperrposition in dem Injektionsverschluss vorhandenen Kräfte (F1, F2, F3) von dem mindestens einen Sperrelement (7; 1007) getragen werden.

3. Autoinjektor nach Anspruch 2, wobei das Trägerplättchen (6; 1006) einen um die Kolbenstange (5; 1005) angeordneten Ring aufweist.

4. Autoinjektor nach einem der vorhergehenden Ansprüche, wobei die Vertiefung (50; 1050) der Kolbenstange (5; 1005) eine geneigte Oberfläche (51) aufweist, die das mindestens eine Sperrelement (7; 1007) radial nach außen spannt.

5. Autoinjektor nach einem der vorhergehenden Ansprüche, wobei die Kraft, die notwendig ist, um das Sperrorgan (23; 1004) aus seiner Verriegelungsposition in seine Entriegelungsposition zu verschieben, mindestens 10%, vorteilhafterweise etwa 5%, der Kraft beträgt, die in der Sperrposition von der Injektionsfeder (8; 1008) auf den Injektionsverschluss ausgeübt wird.

6. Autoinjektor nach einem der vorhergehenden Ansprüche, wobei das Sperrorgan ein Sperrring (23) ist, der in Bezug auf die Steuermuffe (4) axial verschiebbar ist, wobei ein Steuerring (2) mit der Betätigungsmuffe (11) zusammenwirkt, die vorgesehen ist, um mit dem Sperrring (23) zusammenzuwirken, um ihn von seiner Verriegelungsposition in seine Entriegelungsposition zu verschieben.

7. Autoinjektor nach einem der Ansprüche 1 bis 5, wobei das Sperrorgan durch die Steuermuffe (1004) gebildet ist, wobei die Betätigungsmuffe (1011) direkt mit der Steuermuffe (1004) zusammenwirkt, um sie aus ihrer Verriegelungsposition in ihre Entriegelungsposition zu verschieben.

## Claims

1. An autoinjector comprising a body a lower body (10; 1010) and an actuating sleeve (11; 1011), said lower body (10; 1010) receiving a reservoir, said reservoir containing fluid product and comprising a piston and a needle, such as a pre-filled syringe (A), said actuating sleeve (11; 1011) being provided with a contact end designed to make contact with the body of the user, said actuating sleeve (11; 1011) being displaceable in said lower body (10; 1010) between projected positions, in which said actuating sleeve (11; 1011) at least partially projects out of said lower body (10; 1010) and an actuation position, in which said actuating sleeve (11; 1011) is axially displaced towards the interior of said lower body (10; 1010), said autoinjector comprising a piston rod (5; 1005) adapted to cooperate with the piston of said reservoir, said piston rod (5; 1005) being displaceable between a loaded position and an injection position in which said piston rod (5; 1005) has displaced the piston of the reservoir to inject the fluid product through the needle, an injection spring (8; 1008) being provided to stress said piston rod (5; 1005) towards its injection position, the autoinjector comprising an injection lock which blocks said piston rod (5; 1005) in its loaded position, said injection lock comprising a control sleeve (4; 1004), said control sleeve (4; 1004) containing said piston rod (5; 1005) and said injection spring (8; 1008), said piston rod (5; 1005) comprising a radial recess (50; 1050) receiving at least one spherical blocking element (7; 1007) mobile between a blocking position and an unblocking position, said at least one blocking element (7; 1007) being stressed radially towards the exterior by said piston rod (5; 1005) and being held in the blocking position by a blocking member (23; 1004), said blocking member (23; 1004) being displaceable relative to said piston rod (5; 1005) to release said at least one blocking element (7; 1007) and thus unblock said injection lock, allowing said injection spring to displace said piston rod (5; 1005) towards its injection position, said actuating sleeve (11; 1011), in the actuation position, causing displacement of said blocking member (23; 1004) from its locking position towards its unlocking position, to trigger said injection lock **characterized in that** said at least one spherical blocking element (7; 1007) is a ball.

2. The autoinjector according to Claim 1, in which said injection spring (8; 1008) cooperates with a pellet support (6; 1006), stressed axially against said at least one blocking element (7; 1007) in the blocking position, such that all the forces (F1, F2, F3) existing in the injection lock in the blocking position are supported by said at least one blocking element (7; 1007).

3. The autoinjector according to Claim 2, in which said pellet support (6; 1006) comprises a ring arranged around said piston rod (5; 1005).

4. The autoinjector according toany preceding claim, in which said recess (50; 1050) of said piston rod (5; 1005) comprises an inclined surface (51) stressing said at least one blocking element (7; 1007) radially towards the exterior.

5. The autoinjector according to any preceding claim, in which the force necessary to move said blocking member (23; 1004) from its locking position towards its unlocking position represents less than 10%, advantageously around 5%, of the force exerted by said injection spring (8; 1008) on said injection lock in the blocking position.

6. The autoinjector according to any preceding claim, in which said blocking member is a blocking ring (23) displaceable axially relative to the control sleeve (4), a control ring (2) cooperating with said actuating sleeve (11) being provided to cooperate with said blocking ring (23) to move it from its locking position towards its unlocking position.

7. The autoinjector according to any one of claims 1 to 5, in which said blocking member is formed by said control sleeve (1004), said actuating sleeve (1011) cooperating directly with said control sleeve (1004) to move it from its locking position towards its unlocking position.
